(19) Europäisches Patentamt
European Patent Office
Office européen des brevets

(11) EP 3 478 657 B1

(12) **EUROPÄISCHE PATENTSCHRIFT**

(45) Veröffentlichungstag und Bekanntmachung des Hinweises auf die Patenterteilung:
**21.04.2021 Patentblatt 2021/16**

(21) Anmeldenummer: **17739484.8**

(22) Anmeldetag: **21.06.2017**

(51) Int Cl.:
***C07D 209/86*** (2006.01) ***C09K 11/06*** (2006.01)

(86) Internationale Anmeldenummer:
**PCT/EP2017/065225**

(87) Internationale Veröffentlichungsnummer:
**WO 2018/001821 (04.01.2018 Gazette 2018/01)**

(54) **DICARBAZOLBIPHENYLDERIVATE ZUR VERWENDUNG IN OPTOELEKTRONISCHEN VORRICHTUNGEN**

DICARBAZOLBIPHENYLDERIVATIVES FOR USE IN OPTOELECTRONIC DEVICES

DERIVES DE DICARBAZOLEBIPHENYLE ET LEUR UTILISATION POUR DES DISPOSITIFS OPTOELECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.07.2016 DE 102016112078**

(43) Veröffentlichungstag der Anmeldung:
**08.05.2019 Patentblatt 2019/19**

(73) Patentinhaber: **CYNORA GMBH**
**76646 Bruchsal (DE)**

(72) Erfinder:
- **BERGMANN, Larissa**
**76199 Karlsruhe (DE)**
- **DANZ, Michael**
**76344 Eggenstein-Leopoldshafen (DE)**
- **ZINK, Daniel**
**76646 Bruchsal (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2016/178463 CN-A- 105 418 486**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 478 657 B1

**Beschreibung**

Die Erfindung betrifft rein organische Moleküle und deren Verwendung in organischen lichtemittierenden Dioden (OLEDs) und in anderen organischen optoelektronischen Vorrichtungen.

**Beschreibung**

Der vorliegenden Erfindung lag die Aufgabe zu Grunde, Moleküle bereitzustellen, die sich zur Verwendung in optoelektronischen Vorrichtungen eignen.

Die Erfindung stellt eine neue Klasse von organischen Molekülen bereit, die sich zur Verwendung in organischen optoelektronischen Vorrichtungen eignen.

Die erfindungsgemäßen organischen Moleküle sind rein organische Moleküle, weisen also keine Metallionen auf und grenzen sich so von den zur Verwendung in organischen optoelektronischen Vorrichtungen bekannten Metallkomplexverbindungen ab.

Die erfindungsgemäßen organischen Moleküle zeichnen sich durch Emissionen im blauen, himmelblauen oder grünen Spektralbereich aus. Die Photolumineszenzquantenausbeuten der erfindungsgemäßen organischen Moleküle betragen insbesondere 20 % und mehr. Die erfindungsgemäßen Moleküle zeigen insbesondere thermisch aktivierte verzögerte Fluoreszenz (TADF). Die Verwendung der erfindungsgemäßen Moleküle in einer optoelektronischen Vorrichtung, beispielsweise einer organischen lichtemittierenden Diode (OLED), führt zu höheren Effizienzen der Vorrichtung. Entsprechende OLEDs weisen eine höhere Stabilität auf als OLEDs mit bekannten Emittermaterialien und vergleichbarer Farbe.

Unter dem blauen Spektralbereich wird hier der sichtbare Bereich von 430 nm bis 470 nm verstanden. Unter dem himmelblauen Spektralbereich wird hier der Bereich von 470 nm bis 499 nm verstanden. Unter dem grünen Spektralbereich wird hier der Bereich von 500 nm bis 599 nm verstanden. Dabei liegt das Emissionsmaximum im jeweiligen Bereich.

Die organischen Moleküle weisen eine Struktur der Formel I auf oder bestehen aus einer Struktur gemäß Formel I:

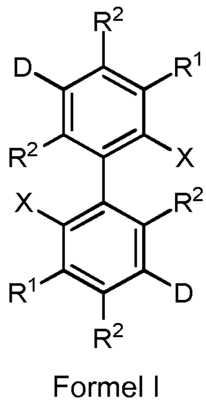

Formel I

mit

X = CN oder $CF_3$,
D =

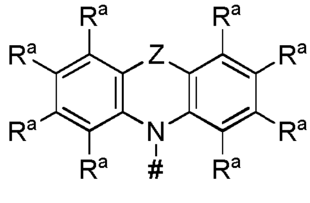

Formel II

# ist Anknüpfungspunkt der Einheit D an einen der in Formel I gezeigten Phenylringe.
Z ist eine direkte Bindung oder ausgewählt aus der Gruppe bestehend aus $CR^3R^4$, $C=CR^3R^4$, C=O, $C=NR^3$, $NR^3$,

O, $SiR^3R^4$, S, S(O), $S(O)_2$.

$R^1$ und $R^2$ ist bei jedem Auftreten gleich oder verschieden H, Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium ersetzt sein können, oder ein aromatisches Ringsystem mit 5 bis 15 aromatischen Ringatomen, das jeweils mit einem oder mehreren Resten $R^6$ substituiert sein kann.

$R^a$, $R^3$ und $R^4$ ist bei jedem Auftreten gleich oder verschieden H, Deuterium, $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^5$ substituiert sein kann, oder eine Aryloxy- oder Heteroaryloxygruppe mit 5 bis 60 aromatischen Ringatomen, die mit einem oder mehreren Resten $R^5$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann.

$R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C{=}CR^6$, C≡C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^6$ substituiert sein kann, oder eine Aryloxy- oder Heteroaryloxygruppe mit 5 bis 60 aromatischen Ringatomen, die mit einem oder mehreren Resten $R^6$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann.

$R^6$ ist bei jedem Auftreten gleich oder verschieden H, Deuterium, OH, $CF_3$, CN, F, Br, I, eine lineare Alkyl-, Alkoxy- oder Thioalkoxygruppe mit 1 bis 5 C-Atomen oder eine lineare Alkenyl- oder Alkinylgruppe mit 2 bis 5 C-Atomen oder eine verzweigte oder cyclische Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen oder eine Aryloxy- oder Heteroaryloxygruppe mit 5 bis 60 aromatischen Ringatomen oder eine Diarylaminogruppe, Diheteroarylaminogruppe oder Arylheteroarylaminogruppe mit 10 bis 40 aromatischen Ringatomen.

Jeder der Reste $R^a$, $R^3$, $R^4$ oder $R^5$ kann auch mit einem oder mehreren weiteren Resten $R^a$, $R^3$, $R^4$ oder $R^5$ ein mono- oder polycyclisches, aliphatisches, aromatisches und/oder benzoannelliertes Ringsystem bilden.

Erfindungsgemäß ist mindestens ein $R^a$ ungleich H und mindestens ein $R^2$ gleich H.

In einer Ausführungsform ist $R^1$ gleich H oder Methyl und $R^2$ gleich H.

In einer weiteren Ausführungsform sind beide X gleich CN.

In einer Ausführungsform der organischen Moleküle sind die beiden Gruppen D identisch, in einer anderen Ausführungsform sind die beiden Gruppen D unterschiedlich.

In einer weiteren Ausführungsform der organischen Moleküle weist eine Gruppe D oder weisen beide Gruppen D eine Struktur der Formel IIa auf bzw. besteht bzw. bestehen aus einer Struktur der Formel IIa:

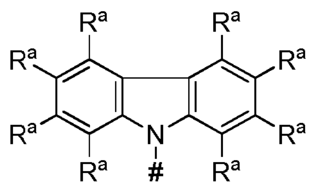

Formel IIa

wobei für # und $R^a$ die oben genannten Definitionen gelten.

In einer weiteren Ausführungsform der erfindungsgemäßen organischen Moleküle weist eine Gruppe D oder weisen beide Gruppen D eine Struktur der Formel IIb, der Formel IIb-2 oder der Formel IIb-3 auf oder besteht bzw.

bestehen daraus:

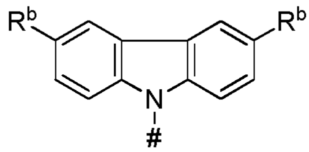
Formel IIb

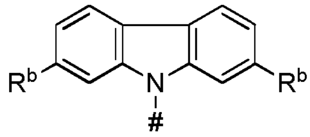
Formel IIb-2

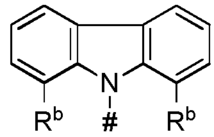
Formel IIb-3

wobei

$R^b$ bei jedem Auftreten gleich oder verschieden ist $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^5$ substituiert sein kann, oder eine Aryloxy- oder Heteroaryloxygruppe mit 5 bis 60 aromatischen Ringatomen, die mit einem oder mehreren Resten $R^5$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann.

Ansonsten gelten die oben genannten Definitionen.

In einer weiteren Ausführungsform der erfindungsgemäßen organischen Moleküle weist eine Gruppe D oder weisen beide Gruppen D eine Struktur der Formel IIc, der Formel IIc-2 oder der Formel IIc-3 auf oder besteht bzw. bestehen daraus:

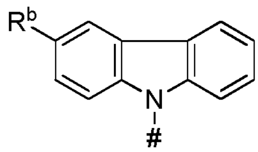
Formel IIc

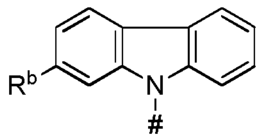
Formel IIc-2

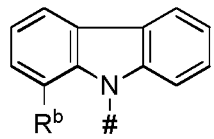
Formel IIc-3

wobei die oben genannten Definitionen gelten.

In einer weiteren Ausführungsform der erfindungsgemäßen organischen Moleküle ist $R^b$ bei jedem Auftreten unabhängig voneinander ausgewählt aus der Gruppe bestehend aus Me, $^i$Pr, $^t$Bu, CN, $CF_3$, Ph, das jeweils mit einem oder mehreren Resten ausgewählt aus Me, $^i$Pr, $^t$Bu, CN, $CF_3$ oder Ph substituiert sein kann, Pyridinyl, Pyrimidinyl, Carbazolyl, das jeweils mit einem oder mehreren Resten ausgewählt aus Me, $^i$Pr, $^t$Bu, CN, $CF_3$, oder Ph substituiert sein kann, und $N(Ph)_2$.

Im Folgenden sind beispielhaft Ausführungsformen der Gruppe D gezeigt:

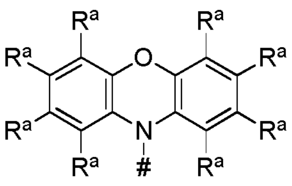
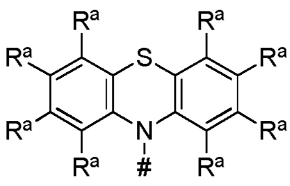
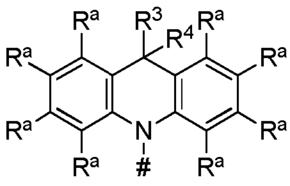
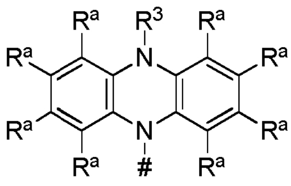

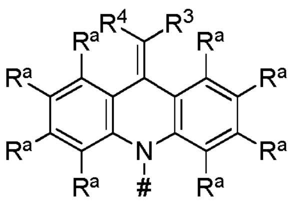
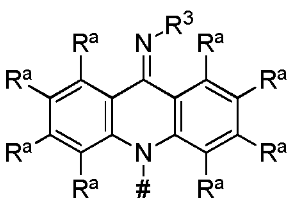
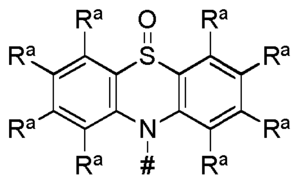

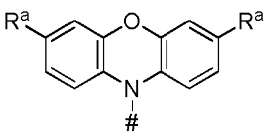
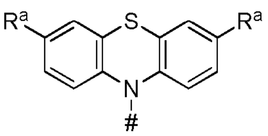
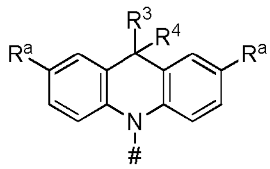
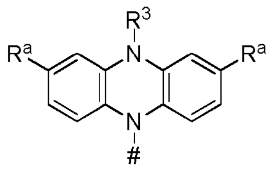

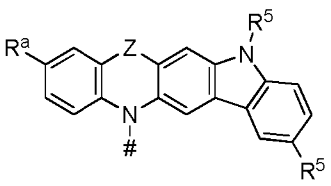
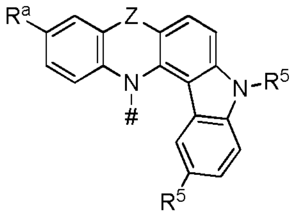
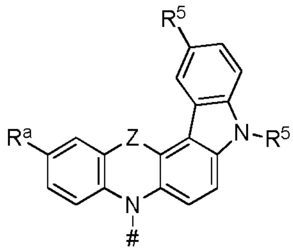

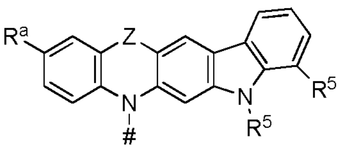
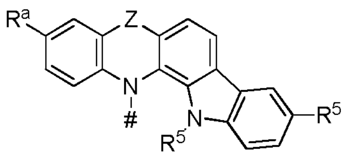
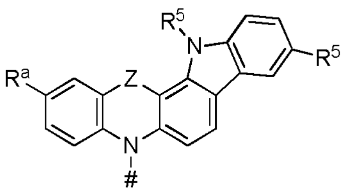

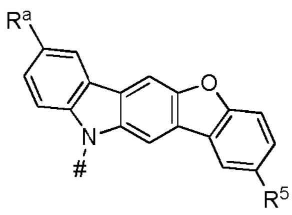
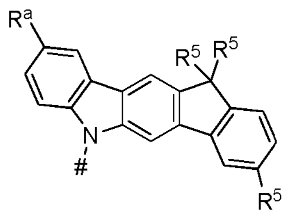
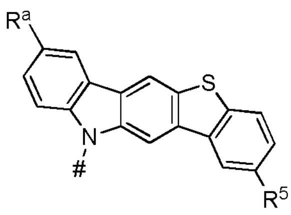

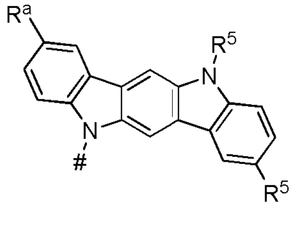

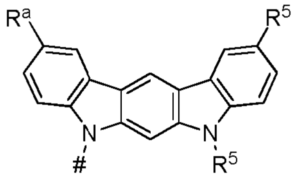 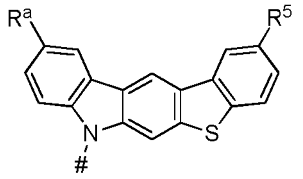 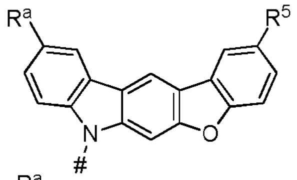

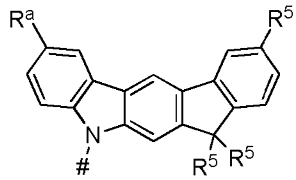 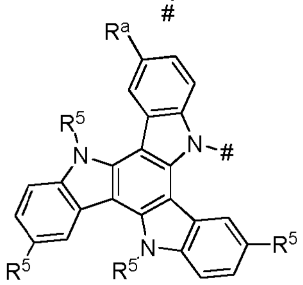

wobei für #, Z, $R^a$ und $R^5$ die oben genannten Definitionen gelten. In einer Ausführungsform ist der Rest $R^5$ bei jedem Auftreten gleich oder verschieden ausgewählt aus der Gruppe bestehend aus H, Methyl, Ethyl, Phenyl und Mesityl.

In einer Ausführungsform ist $R^a$ bei jedem Auftreten gleich oder verschieden ausgewählt aus der Gruppe bestehend aus H, Methyl (Me), i-Propyl ($CH(CH_3)_2$) ($^i$Pr), t-Butyl ($^t$Bu), Phenyl (Ph), CN, $CF_3$ und Diphenylamin ($NPh_2$), wobei mindestens ein $R^a$ ungleich H ist.

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder

Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Eine Ausführungsform der Erfindung betrifft organische Moleküle, welche einen $\Delta E(S_1\text{-}T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen und/oder eine Emissionslebensdauer von höchstens 150 $\mu s$, insbesondere von höchstens 100 $\mu s$, von höchsten 50 $\mu s$, oder von höchstens 10 $\mu s$ aufweisen und/oder eine Hauptemissionsbande mit einer Halbwertsbreite (FWHM) kleiner als 0,55 eV, insbesondere kleiner als 0,50 eV, kleiner als 0,48 eV, oder kleiner als 0,45 eV aufweisen.

Insbesondere weisen die erfindungsgemäßen organischen Moleküle ein Emissionsmaximum zwischen 430 und 520 nm, zwischen 440 und 495 nm oder zwischen 450 und 470 nm auf.

Durch die Lage des Emissionsmaximums in dem genannten Bereich, sind die erfindungsgemäßen organischen Moleküle für die Verwendung in Displays, insbesondere in Ultra High Definition Displays, insbesondere Fernseher (UHDTVs), geeignet. Der Farbraum eines UHDTVs wird nach der sogenannten Rec.2020-Empfehlung (Recommendation ITU-R BT.2020) über die Grundfarben bestimmt. Für Blau liegt der optimale Farbpunkt bei einer $CIE_x$ Koordinate von 0,131 und einer $CIE_y$ Koordinate von 0,046. Dies entspricht bei einer Halbwertsbreite der blauen Emissionsbande von 0 nm einem Emissionsmaximum von 467 nm. Mit zunehmender Halbwertsbreite befindet sich das optimale Emissionsmaximum für die blaue Grundfarbe nach Rec.2020 bei kürzeren Wellenlängen, z.B. bei einer Halbwertsbreite von 35 nm bei 464 nm. Mögliche Emissionsveränderungen durch weitere Komponenten des Displays sind bei diesem Wert nicht berücksichtigt. Somit weist ein idealer blauer Emitter für UHDTV-Anwendungen ein Emissionsmaximum zwischen 450 und 470 nm auf.

Insbesondere weisen die Moleküle einen "blue material index" (BMI), den Quotienten aus der PLQY (in %) und ihrer $CIE_y$-Farbkoordinate des von dem erfindungsgemäßen Molekül emittierten Lichts, von größer 120, insbesondere von größer 200, von größer 250 oder von größer 300 auf.

In einem weiteren Aspekt betrifft die Erfindung ein Verfahren zur Herstellung eines erfindungsgemäßen organischen Moleküls der hier beschriebenen Art (mit einer eventuellen Folgeumsetzung), wobei ein in 6-Position $R^1$-substituiertes und in 3- und 5-Position $R^2$-substituiertes 2-Brom-4-fluorbenzonitril oder ein in 6-Position $R^1$-substituiertes und in 3- und 5-Position $R^2$-substituiertes 2-Brom-4-fluorbenzotrifluorid als Edukt eingesetzt wird.

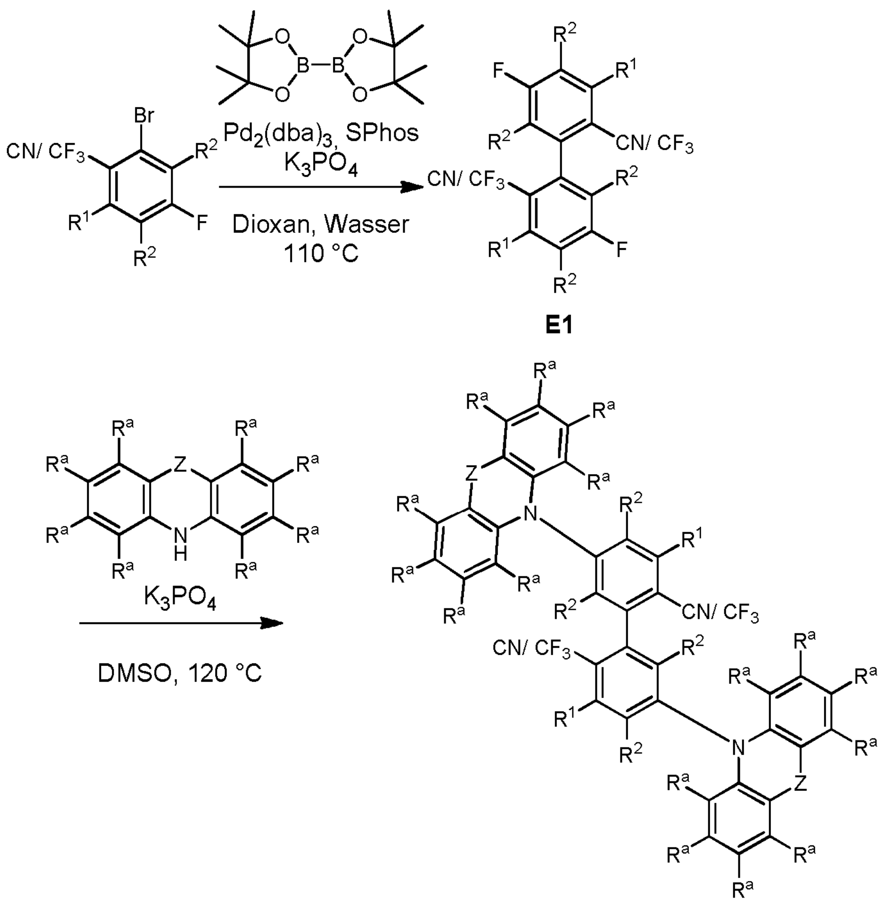

E1

In einer Ausführungsform wird durch Reaktion von in 6-Position $R^1$-substituiertem und in 3- und 5-Position $R^2$-substituiertem 2-Brom-4-fluorbenzonitril oder in 6-Position $R^1$-substituiertem und in 3- und 5-Position $R^2$-substituiertem 2-Brom-4-fluorbenzotrifluorid mit Bis(pinacolato)diboron (4,4,4',4',5,5,5',5'-Octamethyl-2,2'-bi-1,3,2-dioxaborolane) in-situ das entsprechende Kupplungsedukt erzeugt und in einer Palladium-katalysierten Kreuzkupplungsreaktion umgesetzt. Das Produkt wird durch Deprotonierung des entsprechenden Amins und anschließender nukleophiler Substitution der Fluorgruppen erhalten. Hierbei wird ein Stickstoffheterozyklus im Sinne einer nukleophilen aromatischen Substitution mit einem Edukt E1 umgesetzt. Typische Bedingungen beinhalten die Verwendung einer Base wie beispielweise tribasisches Kaliumphosphat oder Natriumhydrid in einem aprotischen polaren Lösungsmittel wie beispielweise Dimetylsulfoxid (DMSO) oder N,N-Dimethylformamid (DMF).

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und
(b) mindestens ein, d. h. ein oder mehrere Emitter- und/oder Hostmaterialien, die von dem erfindungsgemäßen organischen Molekül verschiedenen ist bzw. sind und
(c) optional einem oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)
6. Emissionsschicht bzw. Emitterschicht (emitting layer, EML)
7. Elektronenblockierschicht (electron blocking layer, EBL)
8. Lochtransportschicht (hole transport layer, HTL)
9. Lochinjektionsschicht (hole injection layer, HIL)
10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen. Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Desweiteren können kleine Moleküle verwendet werden (z. B. Kupfer-Phthalocyanin (CuPc z. B. 10 nm dick)) oder Metalloxide wie beispielhaft $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[N,N-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis 100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle

verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

Dem Fachmann ist hierbei bekannt, welche Kombinationen der Materialien für eine optoelektronische Vorrichtung enthaltend ein erfindungsgemäßes organisches Molekül zu nutzen sind.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierende Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)- und Singulett ($S_1$)-Energieniveaus energetisch höher liegen als die Triplett ($T_1$)- und Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED (AMOLED) Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

Durch die nachfolgenden Beispiele wird die Erfindung wird nun näher erläutert, ohne sie dadurch einschränken zu wollen.

**Beispiele**

Allgemeines Syntheseschema

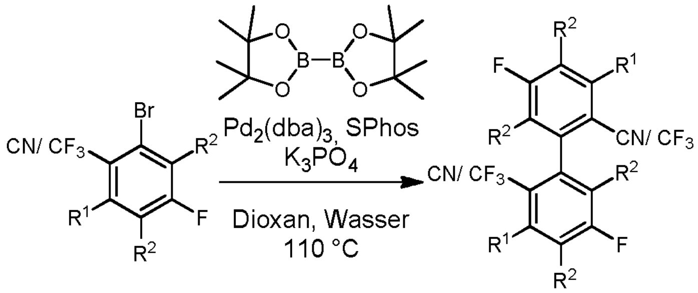


**E1**

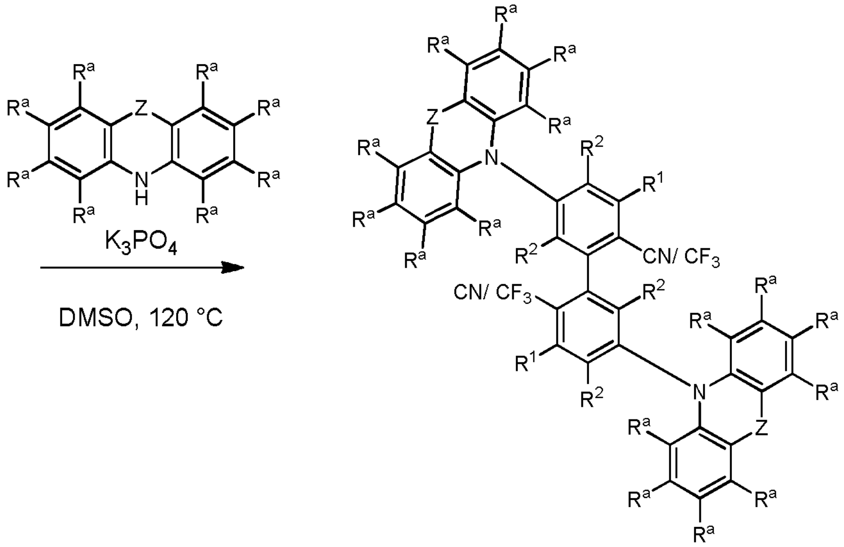


*Allgemeine Synthesevorschrift **AAV1:***

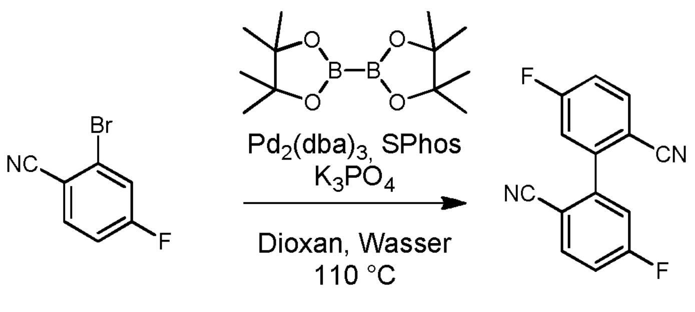


**Z1**

2-Brom-4-fluorbenzonitril (2,00 Äquivalente), Bis(pinacolato)diboron (1,00 Äquivalente), $Pd_2(dba)_3$ (0,01 Äquivalente), SPhos (0,04 Äquivalente) und tribasisches Kaliumphosphat (6,00 Äquivalente) werden unter Stickstoff in einem Dioxan/Wasser-Gemisch (Verhältnis 10:1) bei 110 °C für 16 h gerührt. Die Reaktionsmischung wird mit Wasser gequenscht und die wässrige Phase dann mit Ethylacetat extrahiert. Die vereinigten organischen Phasen werden mit

gesättigter NaCl-Lösung gewaschen, getrocknet über Magnesiumsulfat und das Lösemittel unter vermindertem Druck entfernt. Das Produkt wird anschließend durch Umkristallisation aus Toluol erhalten.

*Allgemeine Synthesevorschrift **AAV2:***

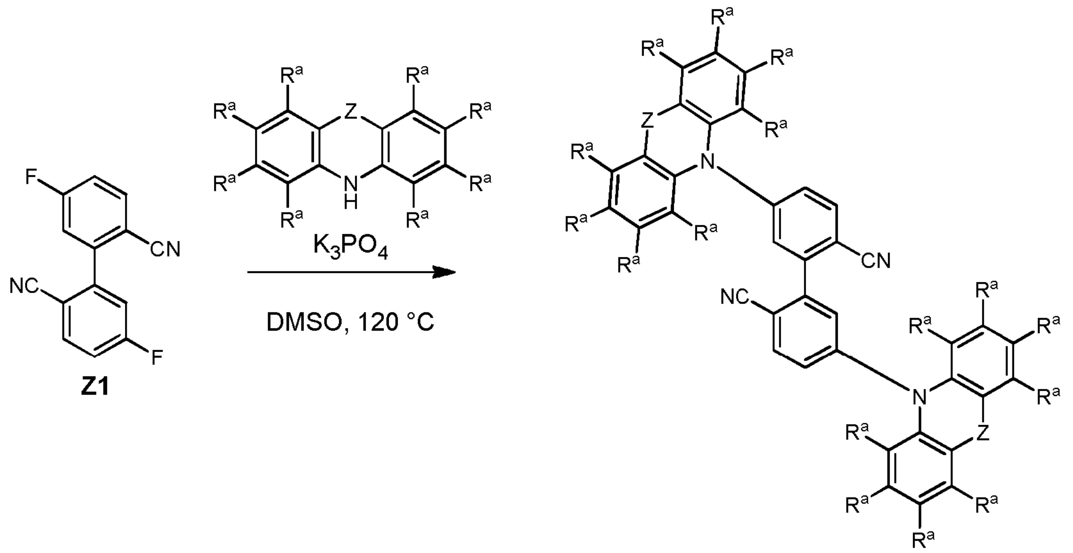


**Z1**

Z1 (1,00 Äquivalente), das entsprechende Donor-Molekül D-H (2,00 Äquivalente) und tribasisches Kaliumphosphat (4,00 Äquivalente) werden unter Stickstoff in DMSO suspendiert und bei 110 °C gerührt (16 h). Anschließend wird die Reaktionsmischung in gesättigte Natriumchlorid-Lösung gegeben und dreimal mit Dichlormethan extrahiert. Die vereinigten organischen Phasen werden zweimal mit gesättigter Natriumchlorid-Lösung gewaschen, getrocknet über Magnesiumsulfat und das Lösemittel anschließend entfernt. Das Rohprodukt wurde schließlich durch Umkristallisation aus Toluol gereinigt. Das Produkt wird als Feststoff erhalten.

*Allgemeine Synthesevorschrift **AAV3:***

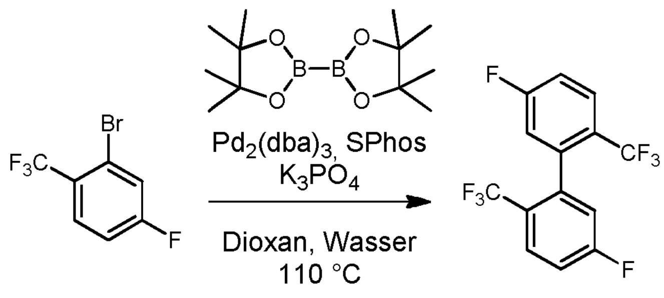


**Z2**

2-Brom-4-fluorbenzotrifluorid (1,00 Äquivalente), Bis(pinacolato)diboron (2,00 Äquivalente), $Pd_2(dba)_3$ (0,01 Äquivalente), SPhos (0,04 Äquivalente) und tribasisches Kaliumphosphat (6,00 Äquivalente) werden unter Stickstoff in einem Dioxan/Wasser-Gemisch (Verhältnis 10:1) bei 110 °C für 16 h gerührt. Anschließend werden die unlöslichen Bestandteile der Reaktionsmischung abfiltriert und mit Dioxan nachgewaschen. Das Lösemittel des Filtrats wird entfernt und der erhaltene Rückstand in Toluol umkristallisiert.

*Allgemeine Synthesevorschrift **AAV4**:*

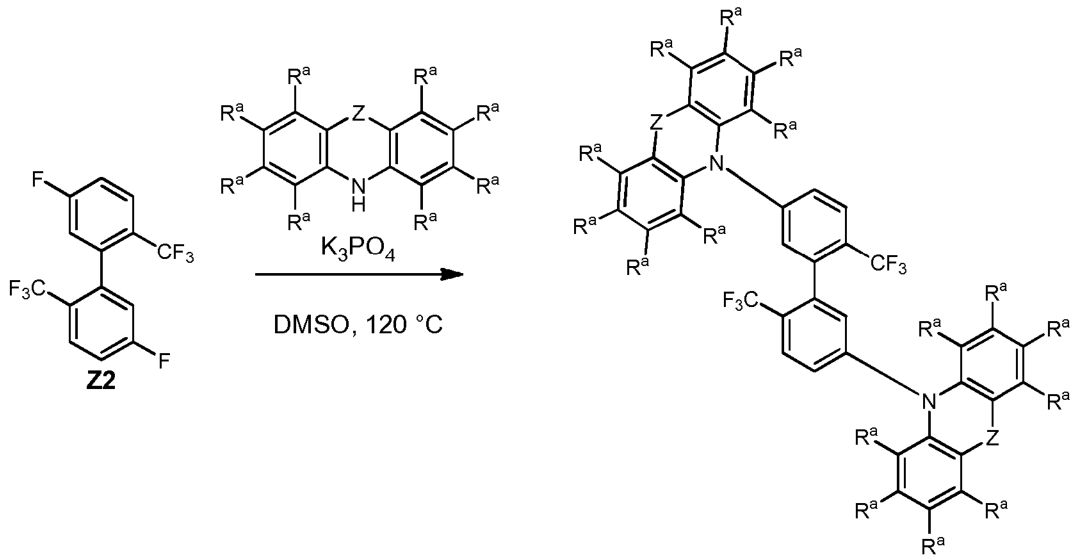


**Z2** (1,00 Äquivalente), das entsprechende Donor-Molekül D-H (2,00 Äquivalente) und tribasisches Kaliumphosphat (4,00 Äquivalente) werden unter Stickstoff in DMSO suspendiert und bei 110 °C gerührt (16 h). Anschließend wird die Reaktionsmischung in gesättigte Natriumchlorid-Lösung gegeben und dreimal mit Dichlormethan extrahiert. Die vereinigten organischen Phasen werden zweimal mit gesättigter Natriumchlorid-Lösung gewaschen, getrocknet über Magnesiumsulfat und das Lösemittel anschließend entfernt. Das Rohprodukt wurde schließlich durch Umkristallisation aus Toluol gereinigt. Das Produkt wird als Feststoff erhalten.

Im speziellen entspricht D-H einem 3,6-substituierten Carbazol (z. B. 3,6-Dimethylcarbazol, 3,6-Diphenylcarbazol, 3,6-Di-tert-butylcarbazol), einem 2,7-substituierten Carbazol (z. B. 2,7-Dimethylcarbazol, 2,7-Diphenylcarbazol, 2,7-Di-tert-butylcarbazol), einem 1,8-substituierten Carbazol (z. B. 1,8-Dimethylcarbazol, 1,8-Diphenylcarbazol, 1,8-Di-tert-butylcarbazol), einem 1-substituierten Carbazol (z. B. 1-Methylcarbazol, 1-Phenylcarbazol, 1-tert-Butylcarbazol), einem 2-substituierten Carbazol (z. B. 2-Methylcarbazol, 2-Phenylcarbazol, 2-tert-Butylcarbazol) oder einem 3-substituierten Carbazol (z. B. 3-Methylcarbazol, 3-Phenylcarbazol, 3-tert-Butylcarbazol).

**Photophysikalische Messungen**

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

*Probenvorbereitung, Film: Spin-Coating*

Gerät: Spin150, SPS euro.

Die Probenkonzentration entsprach 10 mg/ml, angesetzt in Toluol oder Chlorbenzol. Programm: 1) 3 s bei 400 U/min; 2) 20 s bei 1000 U/min bei 1000 Upm/ s. 3) 10s bei 4000 U/min bei 1000 Upm/s. Die Filme wurden nach dem Beschichten für 1 min bei 70 °C an Luft auf einer Präzisionsheizplatte von LHG getrocknet.

Photolumineszenzspektroskopie und TCSPC

Steady-state Emissionsspektroskopie wurde mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" (*Time-correlated single-photon counting,* TCSPC)-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen:

NanoLED 370 (Wellenlänge: 371 nm, Pulsdauer: 1,1 ns)
NanoLED 290 (Wellenlänge: 294 nm, Pulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit ei: Durch den Fit vorhergesagte Größe und $O_i$: gemessenen Größe.

Quanteneffizienzbestimmung

Die Messung der Photolumineszenzquantenausbeute (PLQY) erfolgte mittels eines *Absolute PL Quantum Yield Measurement* C9920-03G-Systems der *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0.

Das Emissionsmaximum wird in nm, die Quantenausbeute Φ in % und die CIE-Farbkoordinaten als x,y-Werte angegeben.

Die Photolumineszenzquantenausbeute wurde nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung:
Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

Herstellung und Charakterisierung von organischen Elektrolumineszenzvorrichtungen aus der Gasphase

Mit den erfindungsgemäßen organischen Molekülen können OLED-Devices mittels Vakuum-Sublimationstechnik erstellt werden.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, den Elektrolumineszenzspektren und dem Strom aufgenommen.

Beispiel 1

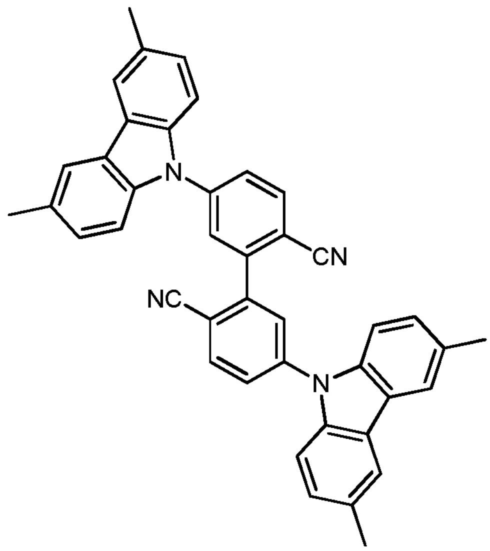

Beispiel 1 wurde nach AAV 1 (Ausbeute 72 %) und AAV2 (Ausbeute 78 %) hergestellt.
$^1$H NMR (500 MHz, Chloroform-d) δ 8.07 (d, 2H, Ar-H), 7.90-7.86 (m, 6H, Ar-H), 7.85 (dd, 2H, Ar-H), 7.56 (d, 4H, Ar-H), 7.27-7.25 (m, 4H, Ar-H), 2.53 (s, 12H, C$H_3$) ppm.

Figur 1 zeigt das Emissionsspektrum von Beispiel 1 (10 % in PMMA). Das Emissionsmaximum liegt bei 448 nm. Die Photolumineszenzquantenausbeute (PLQY) beträgt 68 % und die Halbwertsbreite beträgt 0,45 eV. Die $CIE_x$-Farbkoordinate beträgt 0,16 und die $CIE_y$-Farbkoordinate beträgt 0,13.

**Beispiel 2**

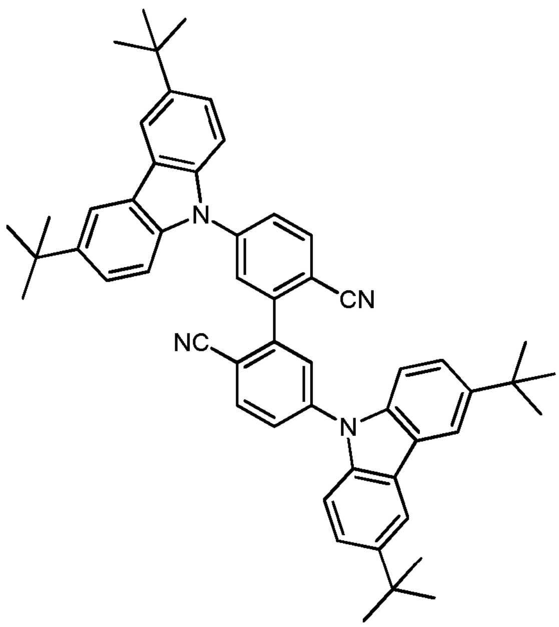

Beispiel 2 wurde nach AAV 1 (Ausbeute 72 %) und AAV2 (Ausbeute 96 %) hergestellt.

Dünnschichtchromatografie: $R_f$ = 0,66 (Cyclohexan/Ethylacetat 5:1)
Figur 2 zeigt das Emissionsspektrum von Beispiel 2 (10 % in PMMA). Das Emissionsmaximum liegt bei 442 nm. Die Photolumineszenzquantenausbeute (PLQY) beträgt 69 % und die Halbwertsbreite beträgt 0,47 eV. Die $CIE_x$-Farbkoordinate beträgt 0,16 und die $CIE_y$-Farbkoordinate beträgt 0,10.

**Beispiel 3**

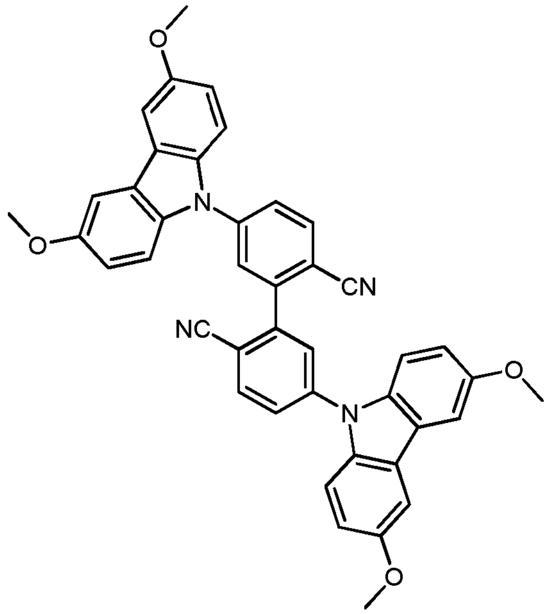

Beispiel 3 wurde nach AAV1 (Ausbeute 72 %) und AAV2 (Ausbeute 33 %) hergestellt.

Figur 3 zeigt das Emissionsspektrum von Beispiel 3 (10 % in PMMA). Das Emissionsmaximum liegt bei 487 nm. Die Photolumineszenzquantenausbeute (PLQY) beträgt 58 % und die Halbwertsbreite beträgt 0,48 eV. Die $CIE_x$-Farbkoordinate beträgt 0,21 und die $CIE_y$-Farbkoordinate beträgt 0,35.

**Beispiel 4**

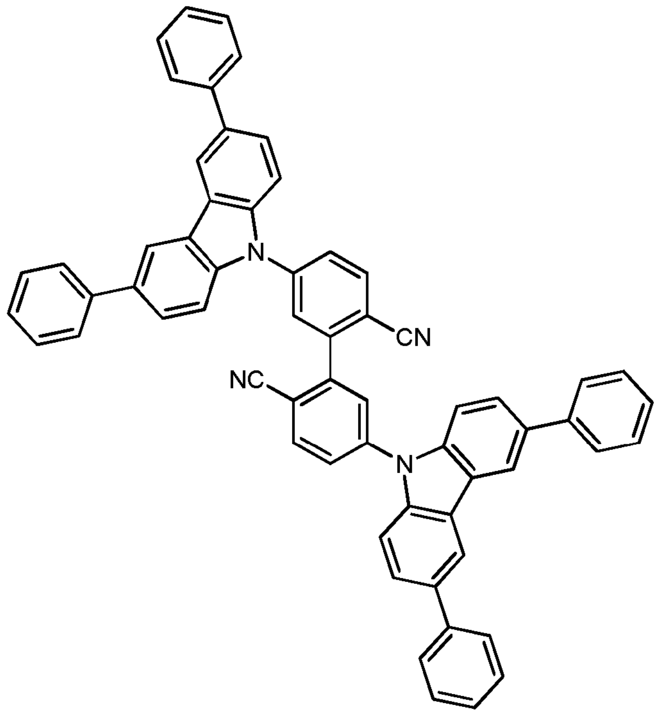

Beispiel **4** wurde nach AAV1 (Ausbeute 72 %) und AAV2 hergestellt.

Dünnschichtchromatografie: $R_f$ = 0,19 (Cyclohexan/Ethylacetat 5:1)

Figur 4 zeigt das Emissionsspektrum von Beispiel **4** (10 % in PMMA). Das Emissionsmaximum liegt bei 444 nm. Die Photolumineszenzquantenausbeute (PLQY) beträgt 34 % und die Halbwertsbreite beträgt 0,52 eV. Die $CIE_x$-Farbkoordinate beträgt 0,19 und die $CIE_y$-Farbkoordinate beträgt 0,17.

**Beispiel 5**

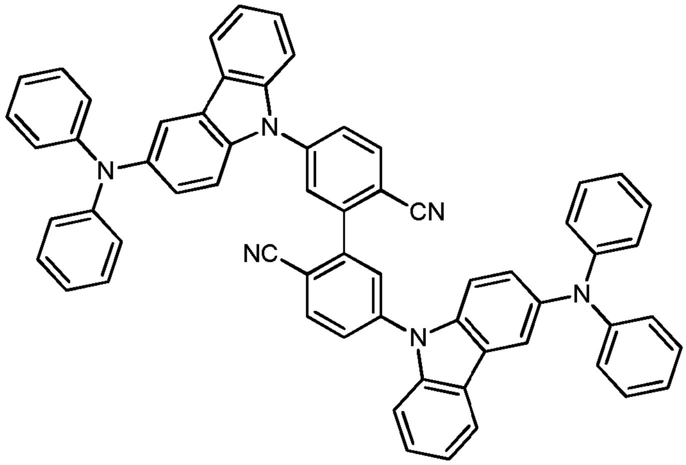

Beispiel 5 wurde nach AAV1 (Ausbeute 72 %) und AAV2 hergestellt.

Dünnschichtchromatografie: $R_f$ = 0,56 (Cyclohexan/Ethylacetat 5:1)

Figur 5 zeigt das Emissionsspektrum von Beispiel **5** (10 % in PMMA). Das Emissionsmaximum liegt bei 509 nm. Die Photolumineszenzquantenausbeute (PLQY) beträgt 57 % und die Halbwertsbreite beträgt 0,51 eV. Die Emissionsabklingzeit wurde mit 21,6 μs bestimmt. Die $CIE_x$-Farbkoordinate beträgt 0,27 und die $CIE_y$-Farbkoordinate beträgt 0,46.

Beispiel 6

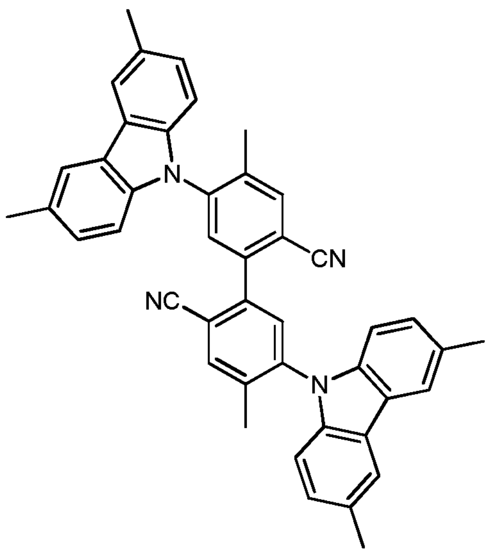

Beispiel **6** wurde mit analog der in AAV1 und AAV2 beschriebenen Reaktionsbedingungen hergestellt, wobei 2-Brom-4-fluor-3-methylbenzonitril statt 2-Brom-4-fluorbenzonitril als Edukt eingesetzt wurde.

$^1H$ NMR (500 MHz, Chloroform-d) δ 7.95-7.93 (m, 6H, Ar-H), 7.71 (s, 2H, Ar-H), 7.28-7.21 (m, 4H, Ar-H), 7.06 (bs, 4H, Ar-H), 2.57 (s, 12H, $CH_3$), 2.16 (s, 6H, $CH_3$) ppm.

Figur 6 zeigt das Emissionsspektrum von Beispiel **6** (10 % in PMMA). Das Emissionsmaximum liegt bei 434 nm. Die Photolumineszenzquantenausbeute (PLQY) beträgt 35 % und die Halbwertsbreite beträgt 0,49 eV.

**Beispiel D1**

Beispiel **2** wurde in einem OLED-Bauteil (Bauteil X1) mit folgendem Aufbau getestet (Anteil des erfindungsgemäßen Moleküls an der Emissionsschicht ist in Massenprozent angegeben, Substrat Glass):

| **Schicht** | | **Dicke** | |
|---|---|---|---|
| 10 | | 100 nm | Al |
| 9 | | 2 nm | Liq |
| 8 | | 30 nm | TPBi |
| 7 | | 10 nm | DPEPO |
| 6 | | 20 nm | 2 (30%):DPEPO |
| 5 | | 10 nm | CzSi |
| 4 | | 20 nm | TCTA |
| 3 | | 50 nm | NPB |
| 2 | | 20 nm | m-MTDATA |
| 1 | | 120 nm | ITO |

Folgende Werte wurden bestimmt:

| | |
|---|---|
| Emissionsmaximum: | 441 nm |
| Maximale Leistungseffizienz: | 6,0 ± 1,7 lm/W |
| Maximale Stromeffizienz: | 12,4 ± 3,3 cd/A |
| CIE: | CIEx: 0,16<br>CIEy: 0,13 at 14 V |
| Maximale Externe Quantenausbeute (EQE): | 11,3 ± 2,7 % |

Weitere Beispiele organischer Moleküle mit einer Struktur gemäß Formel I:

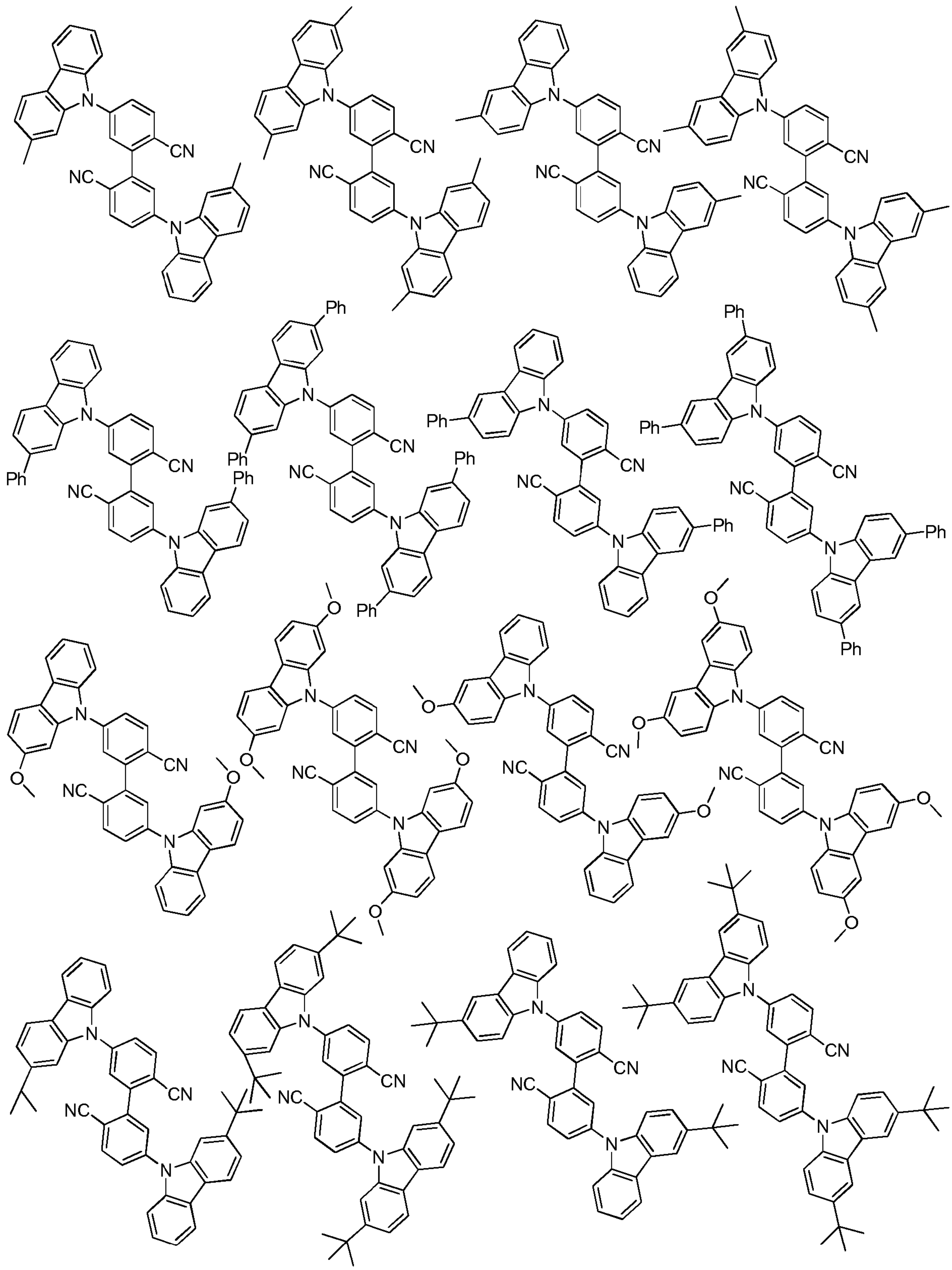

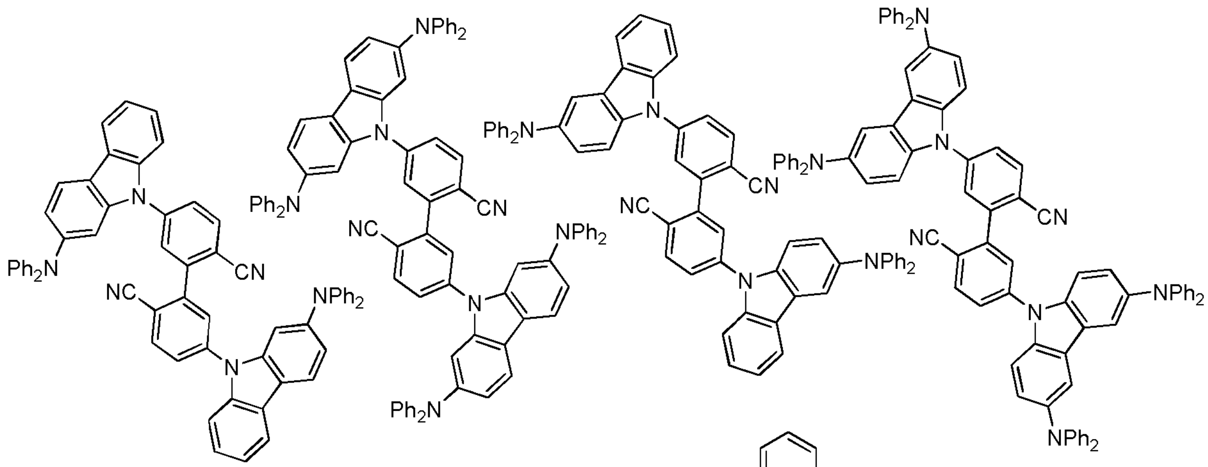

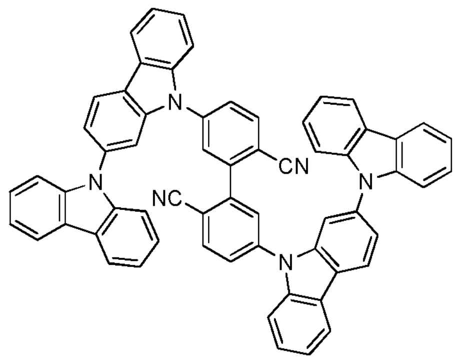

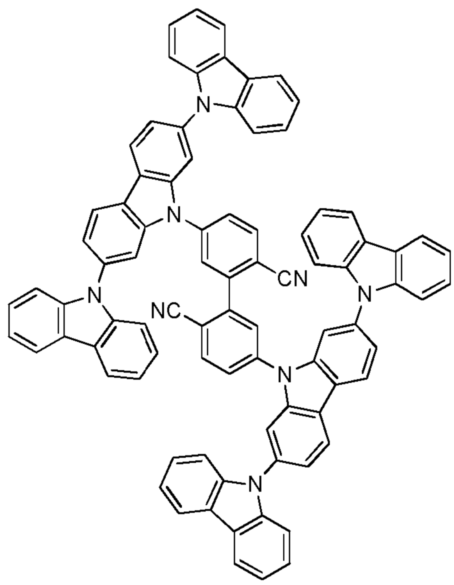

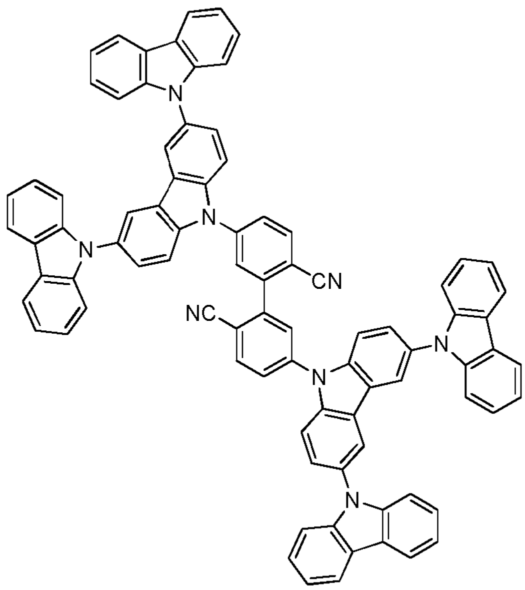

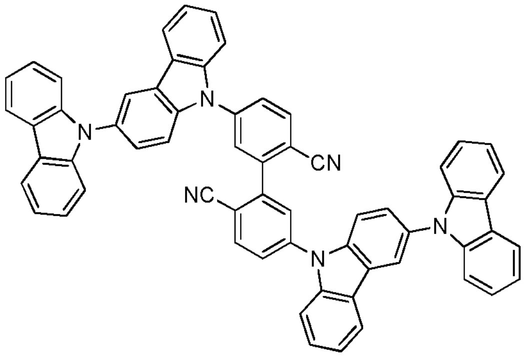

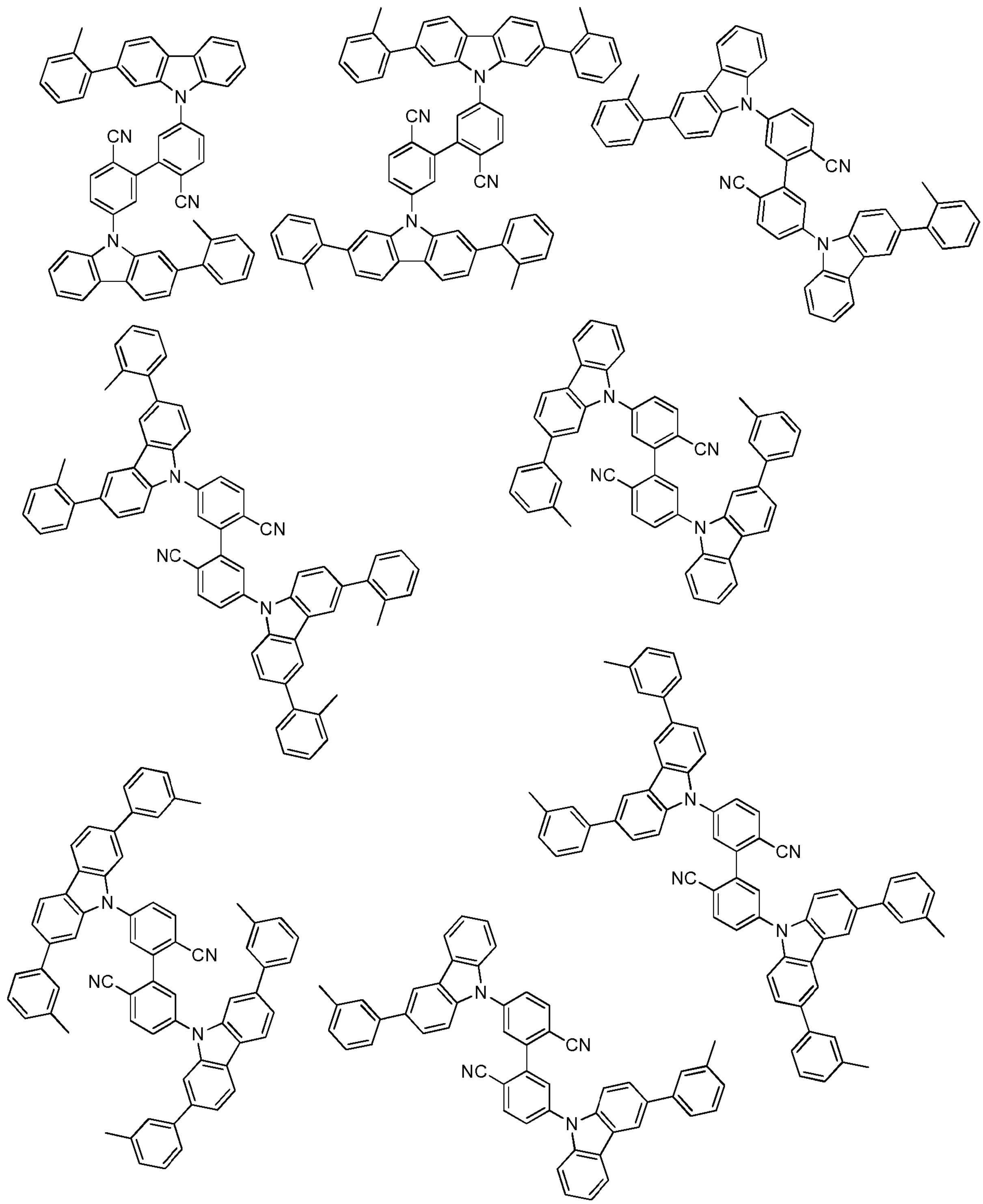

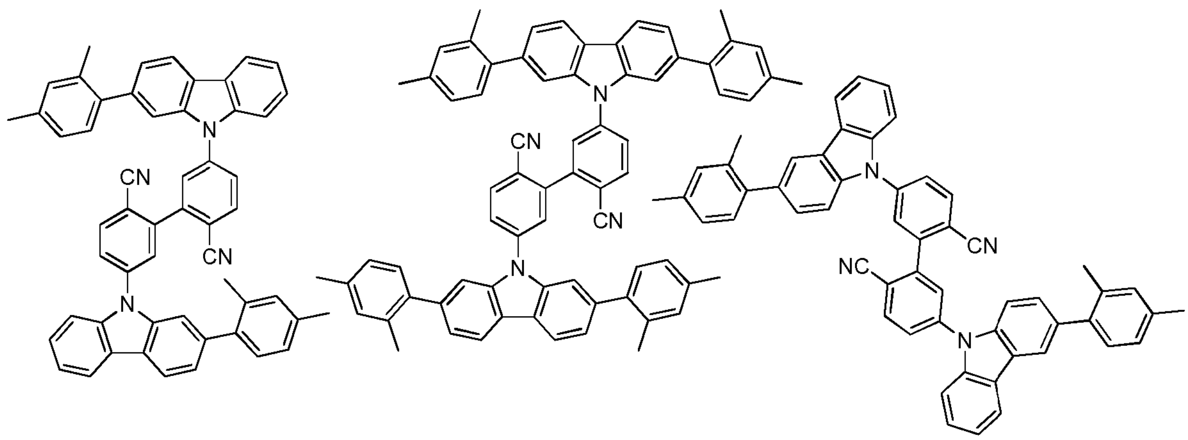

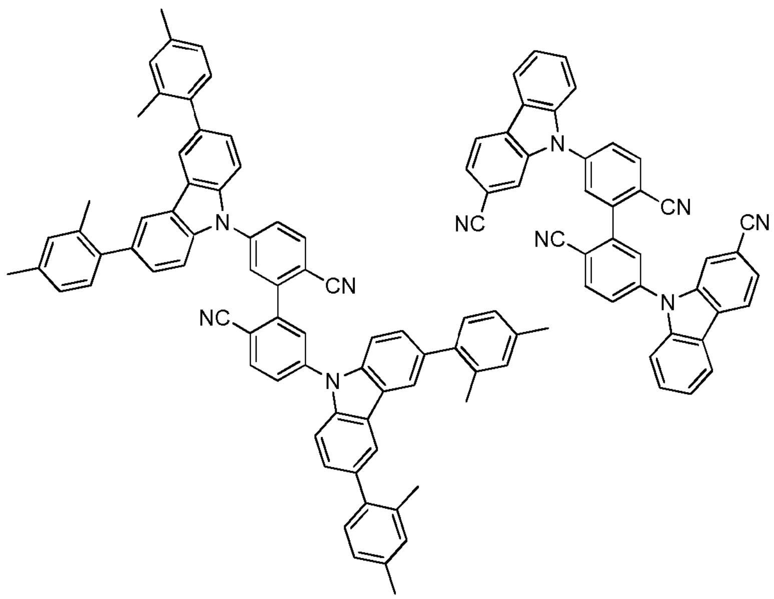

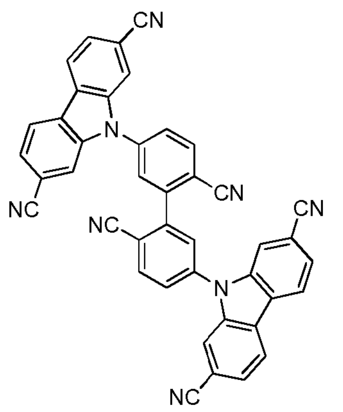

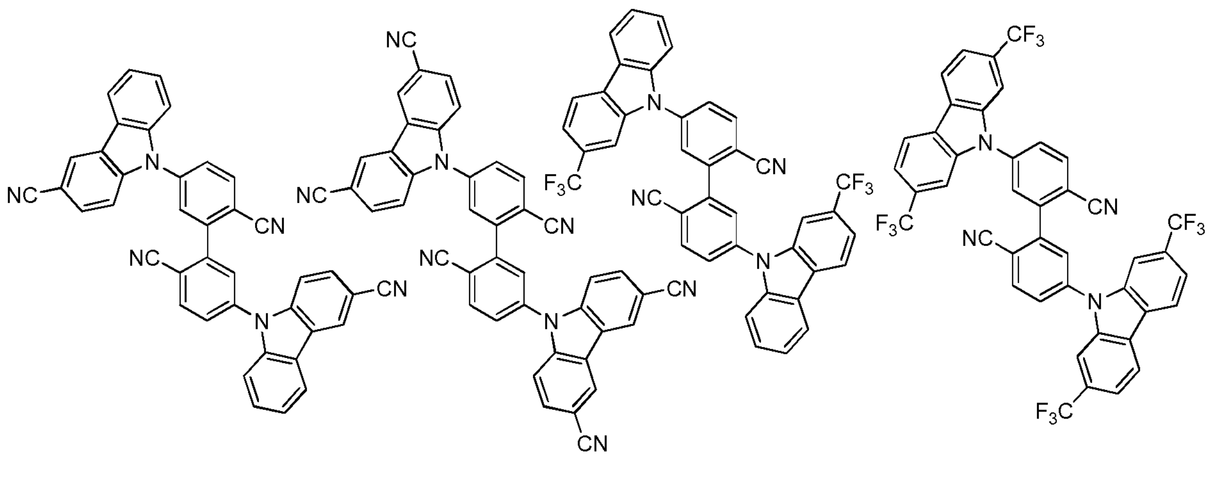

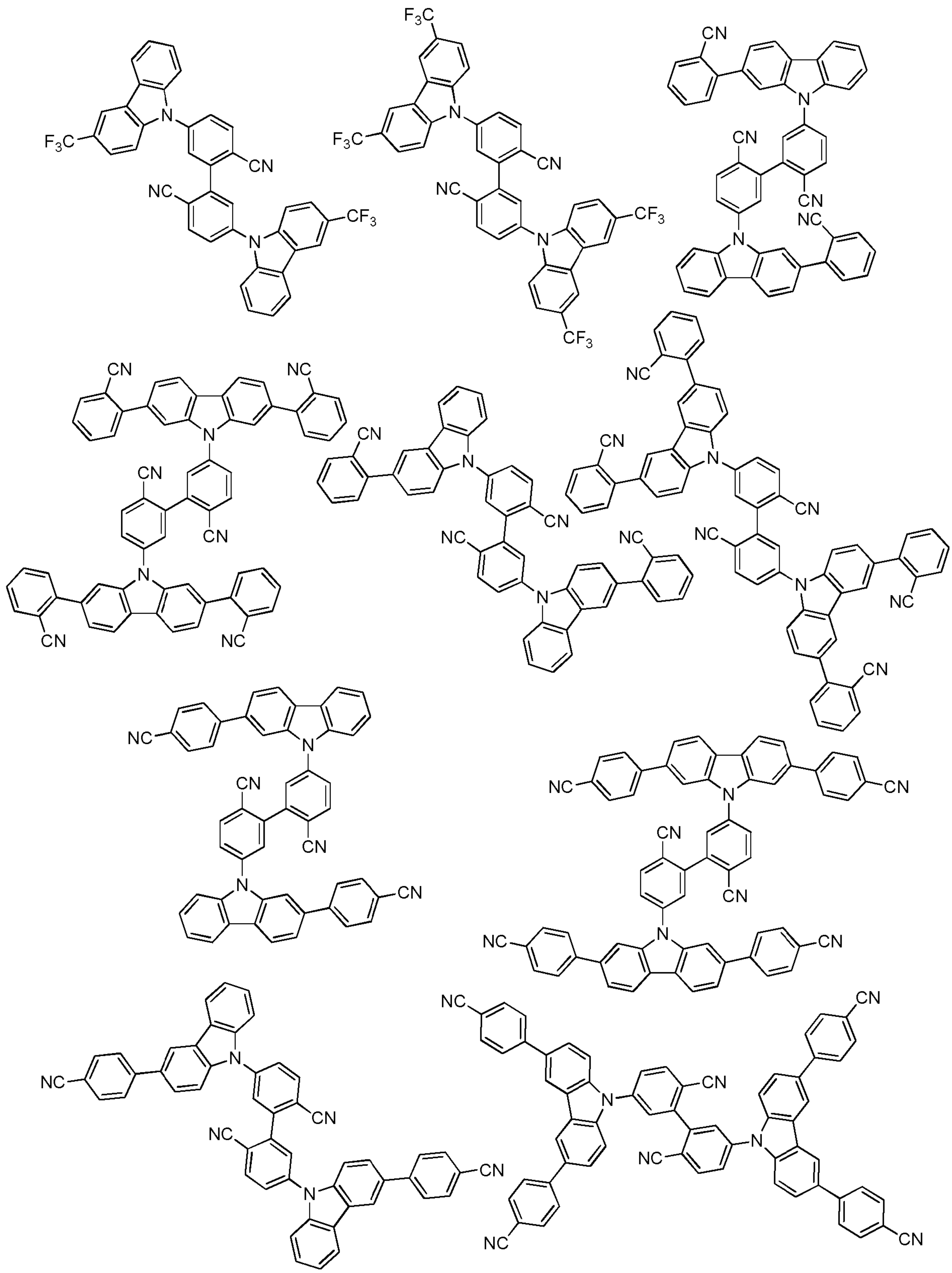

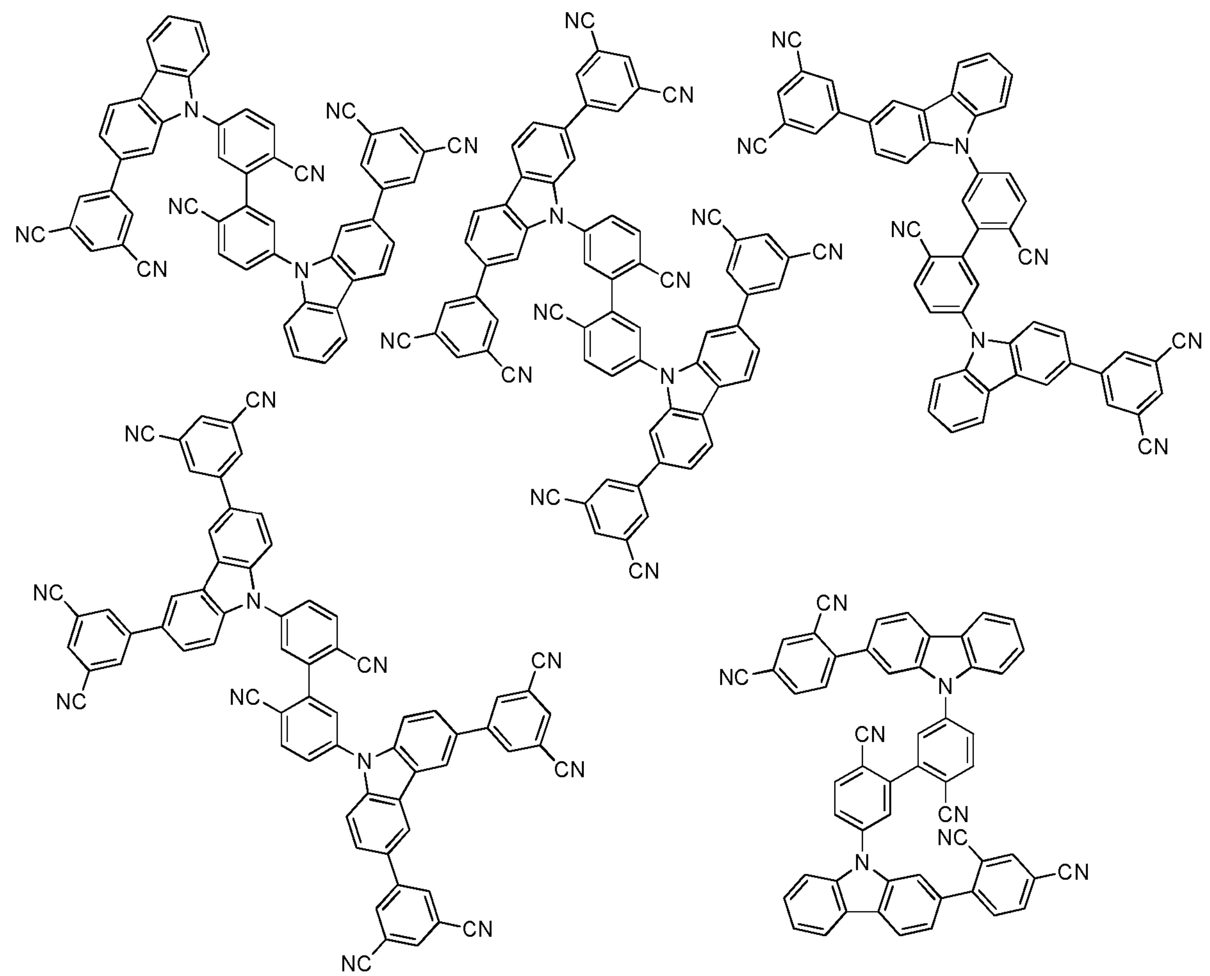

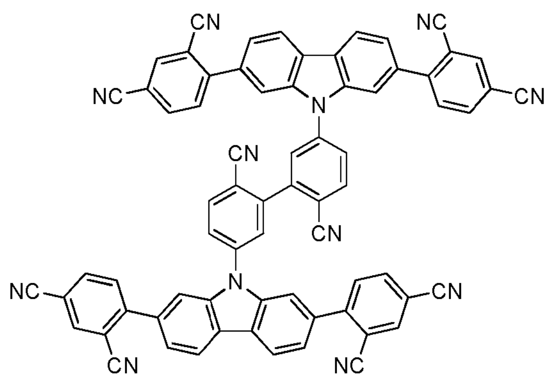

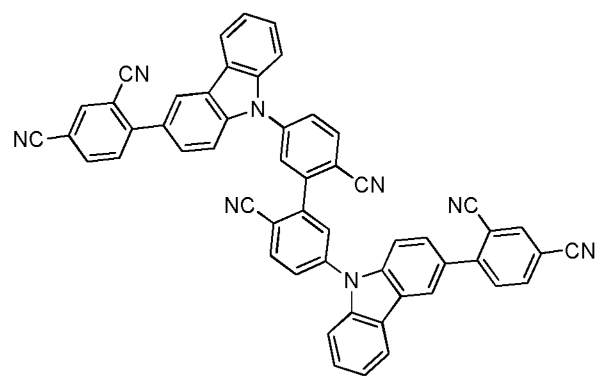

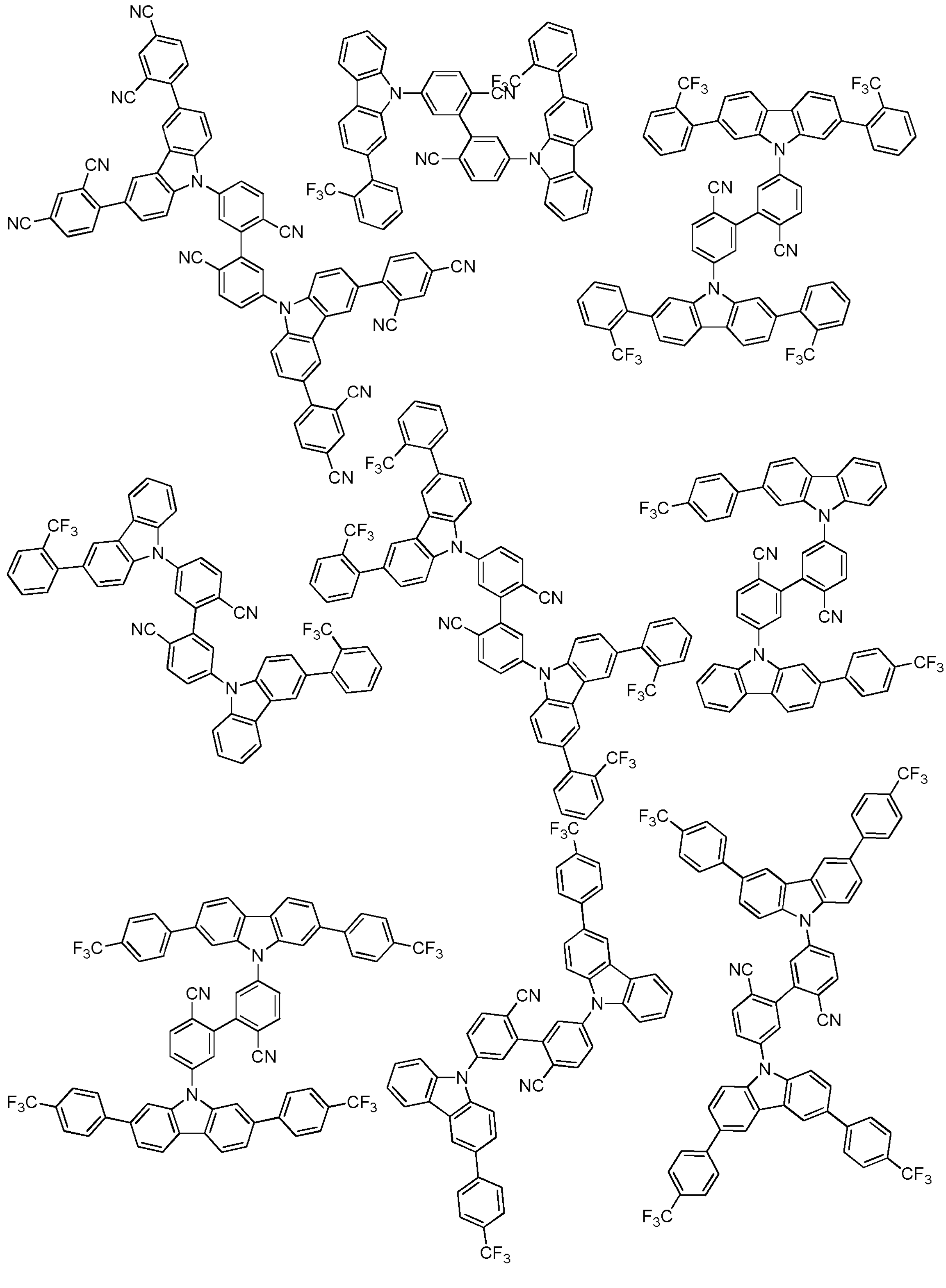

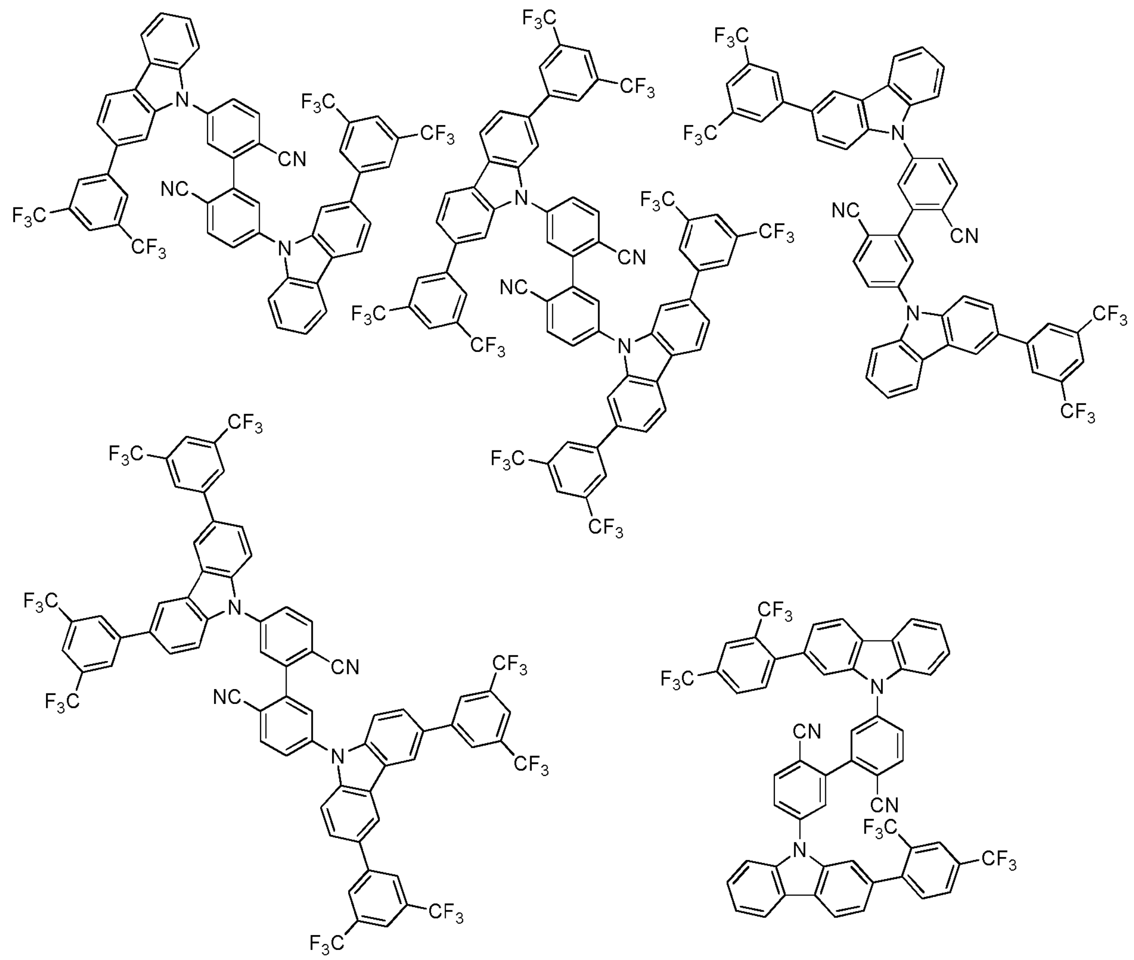

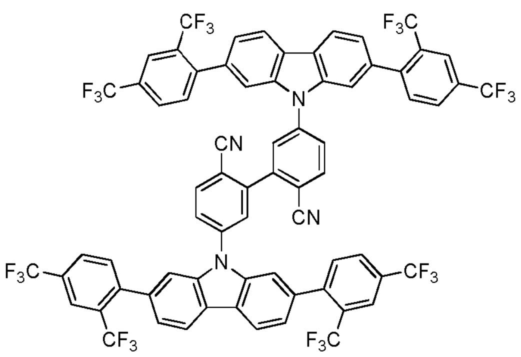

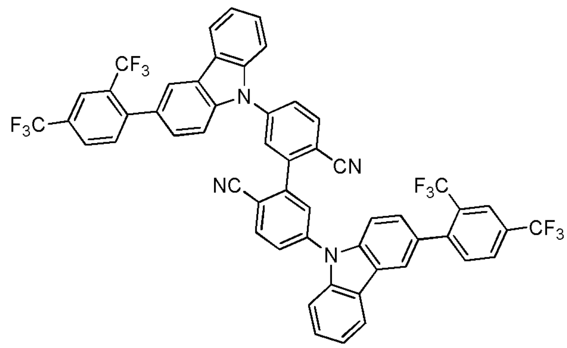

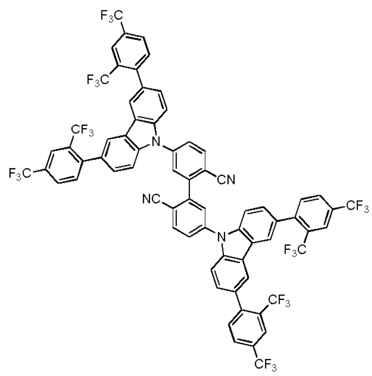

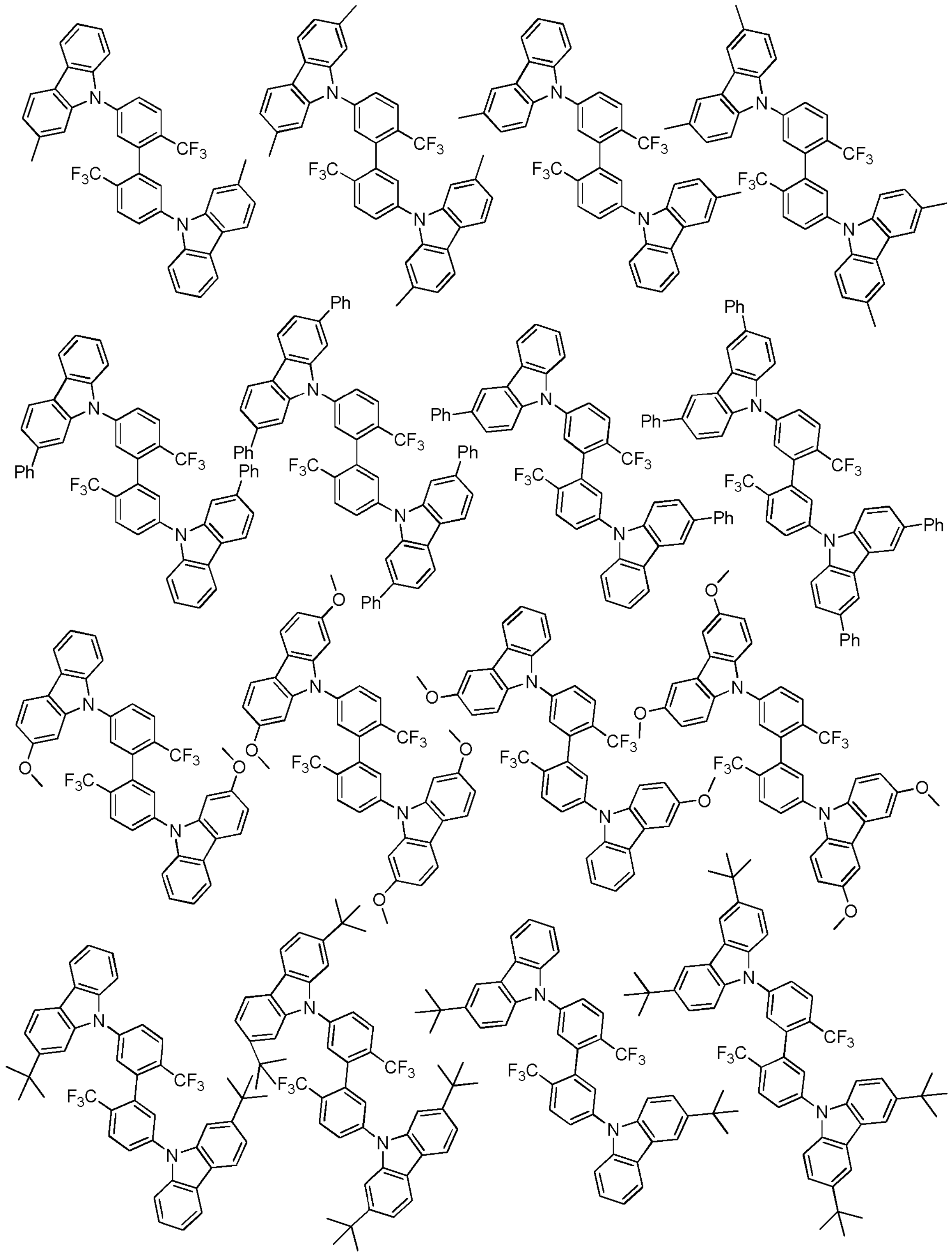

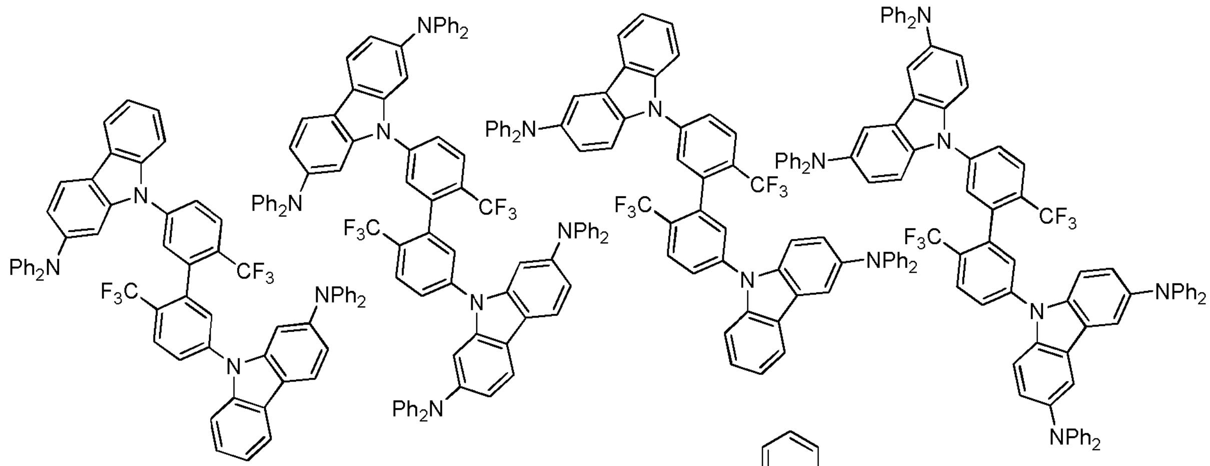

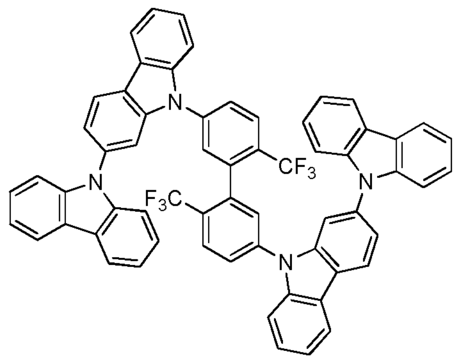

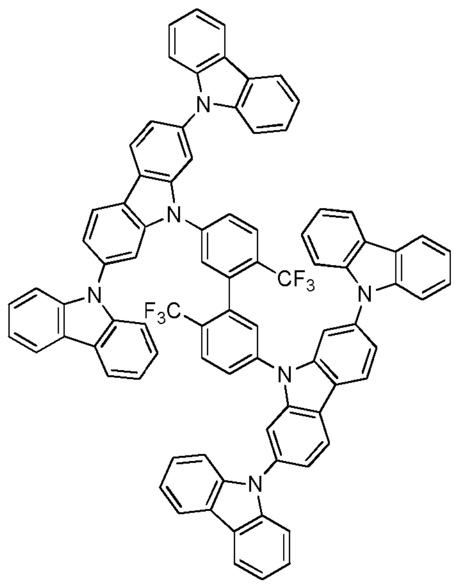

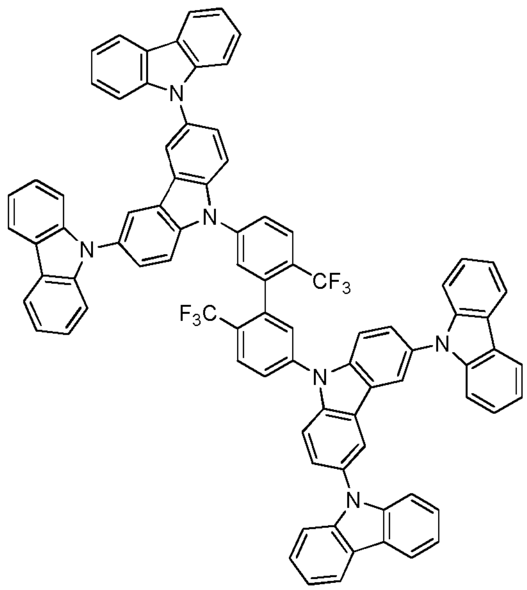

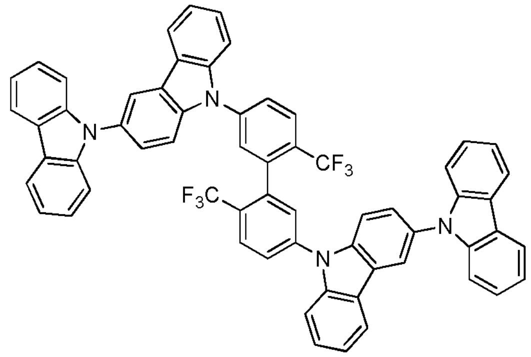

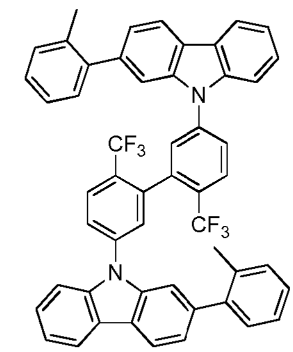

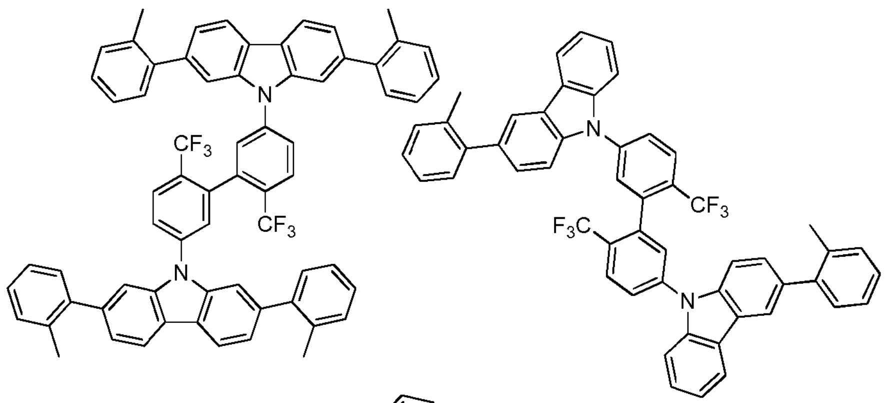

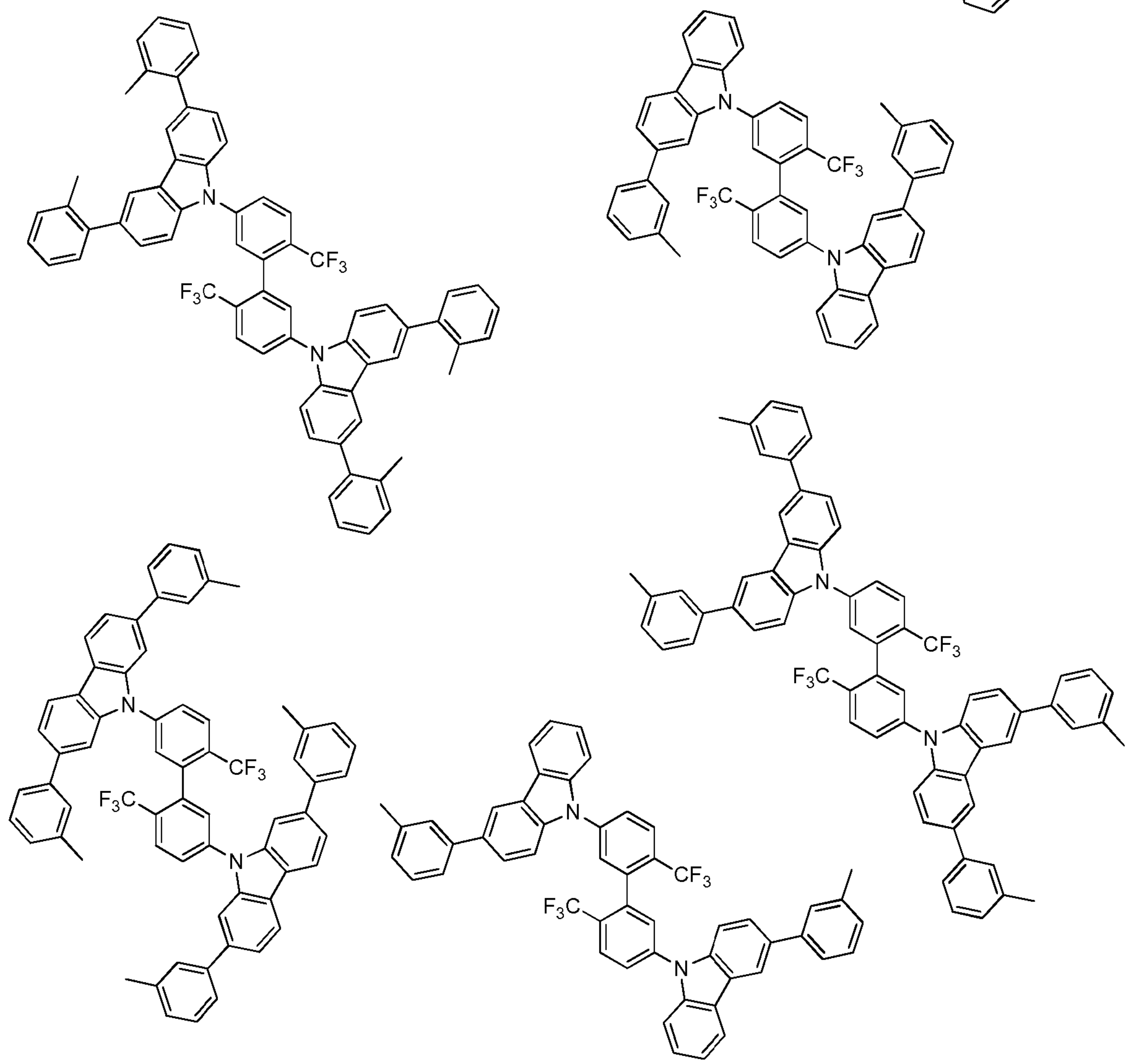

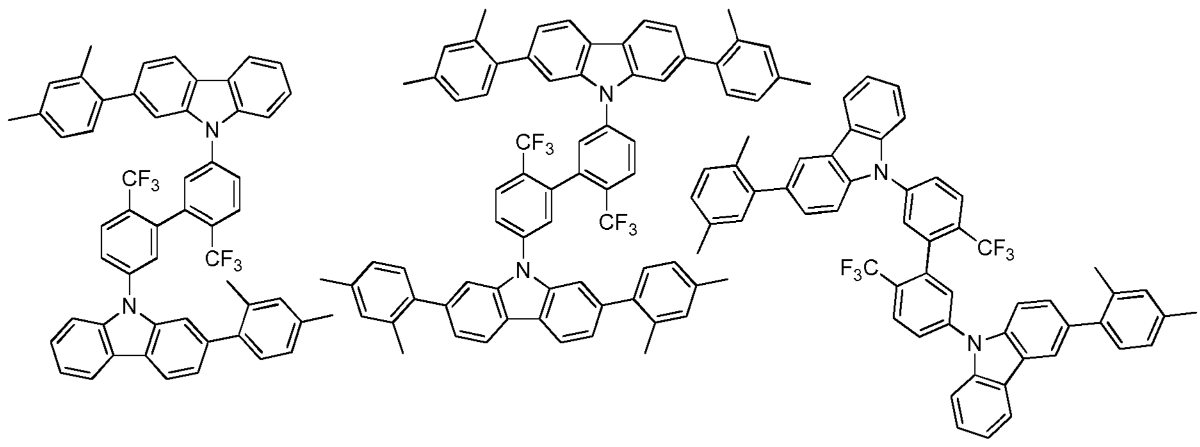

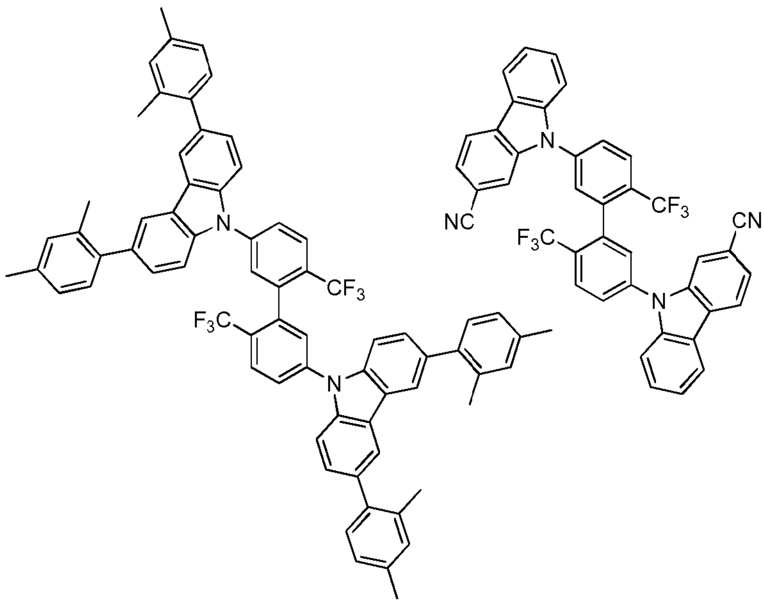

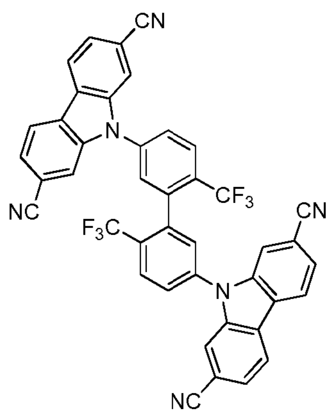

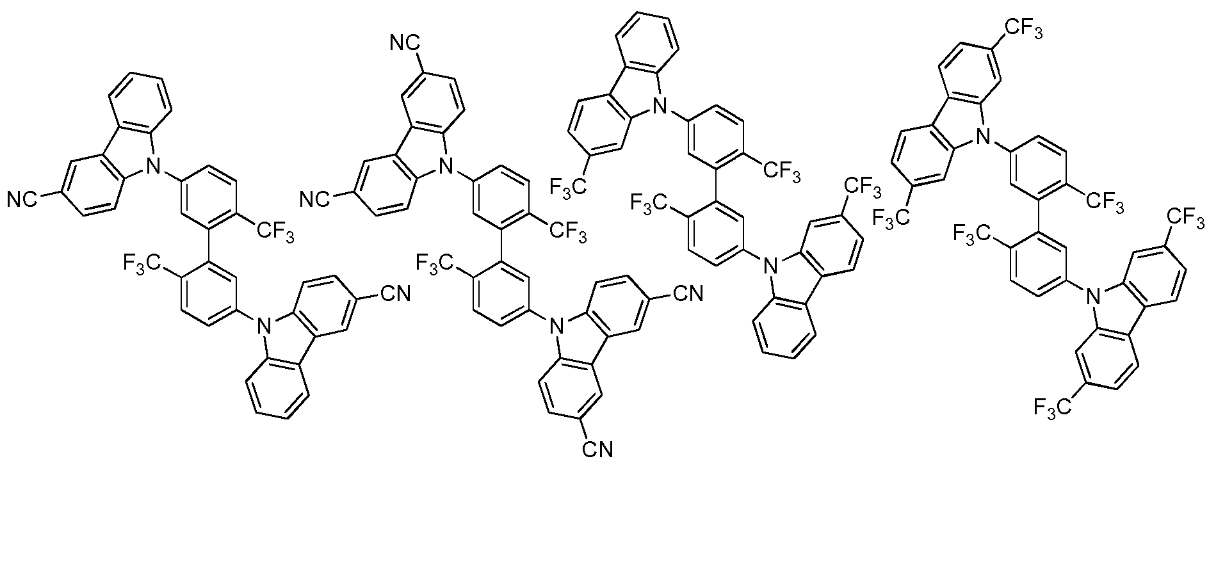

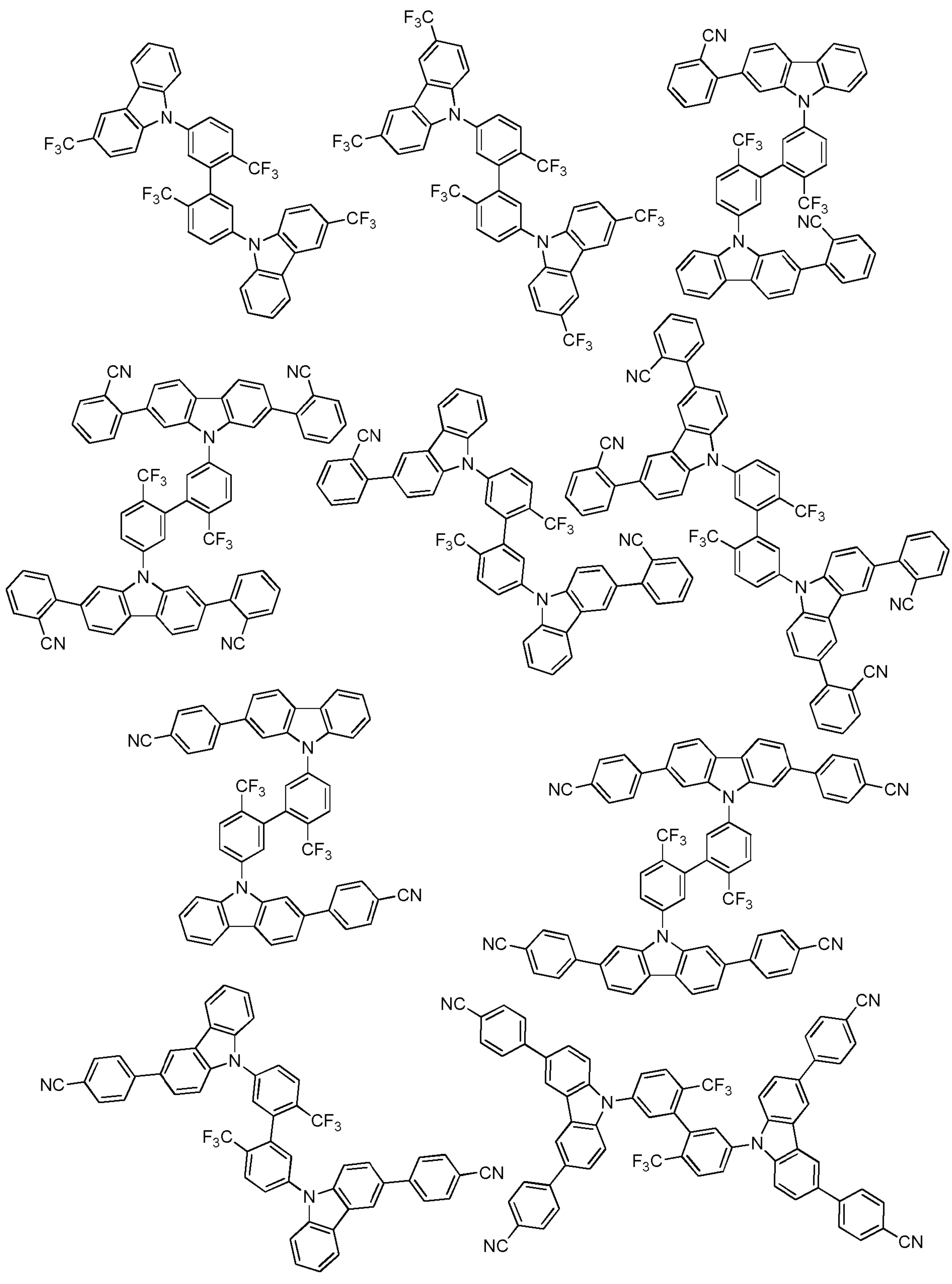

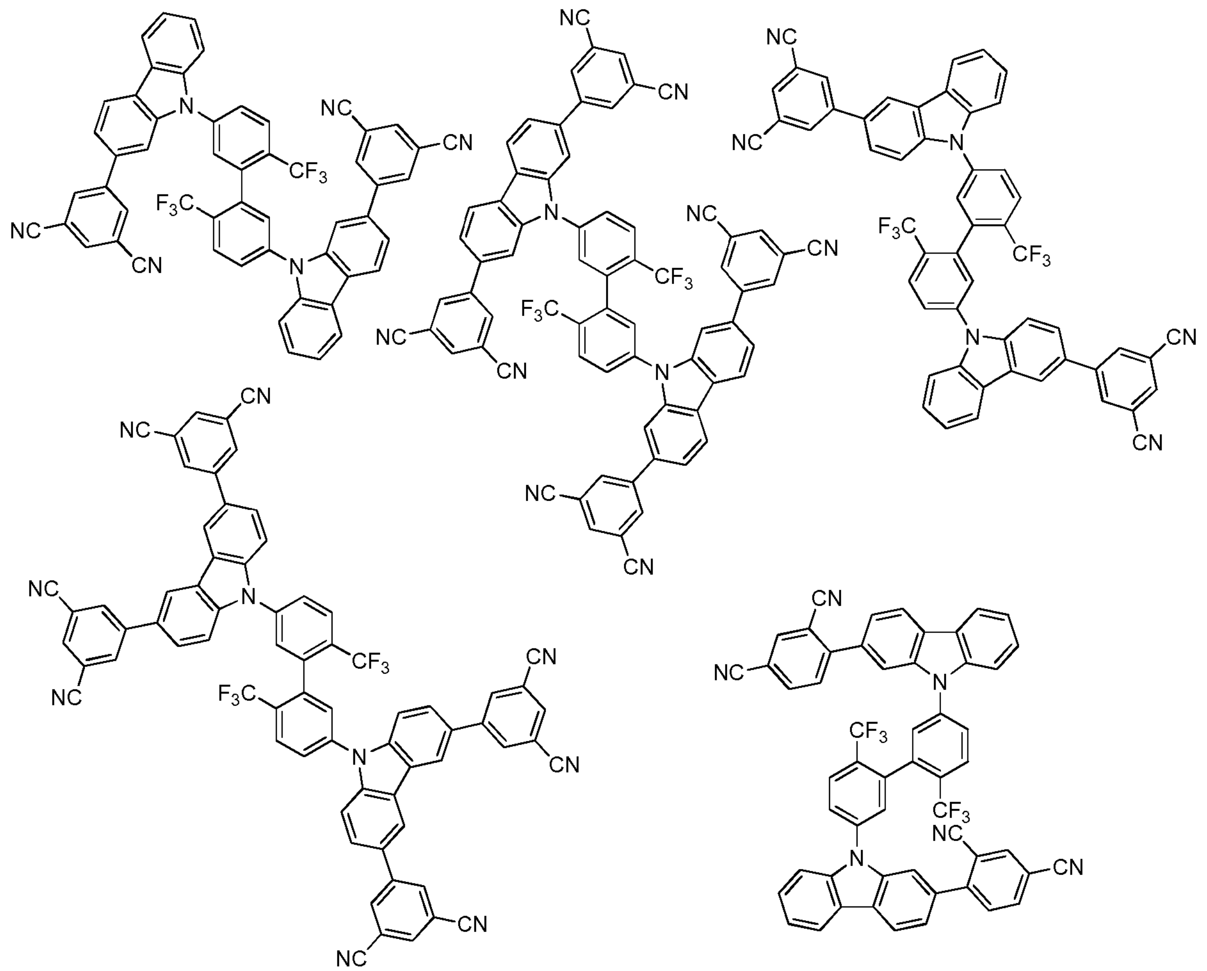

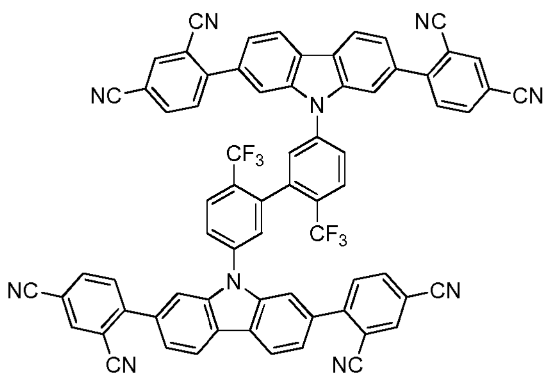

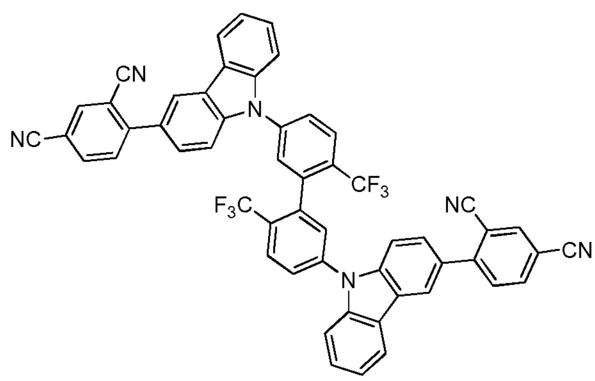

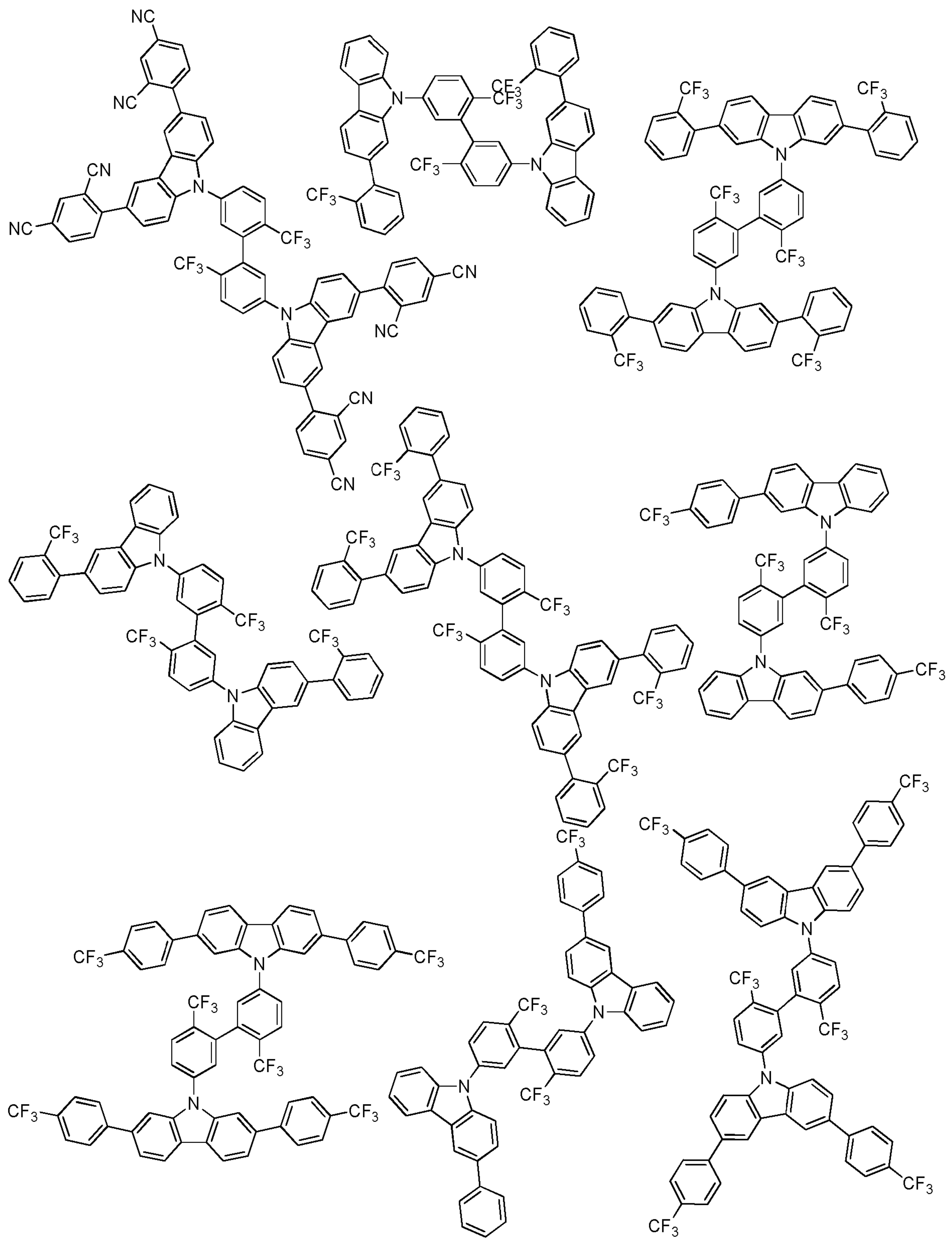

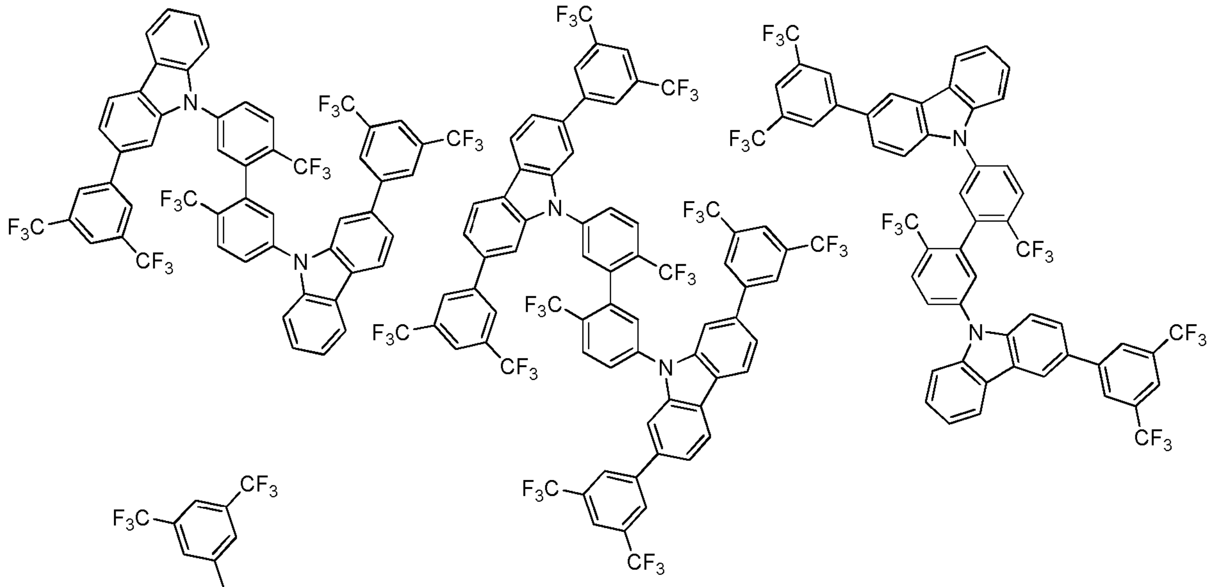

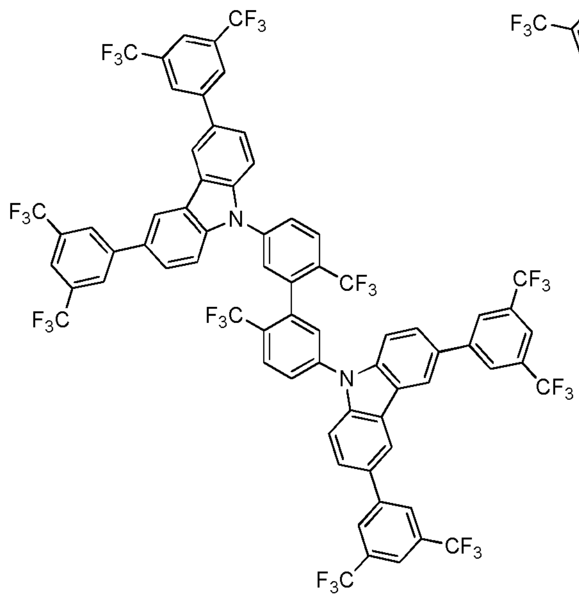

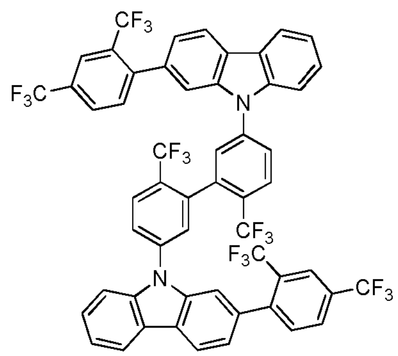

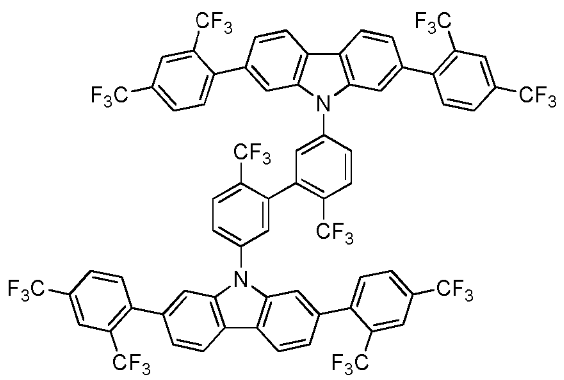

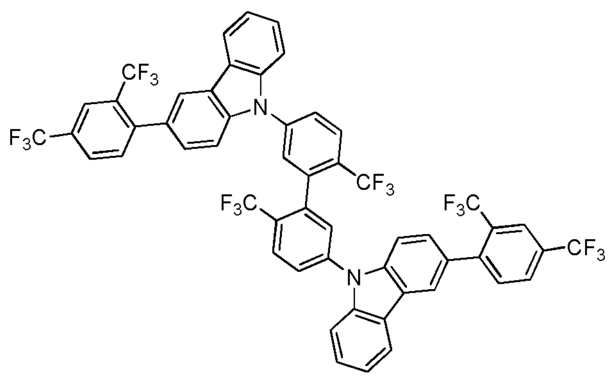

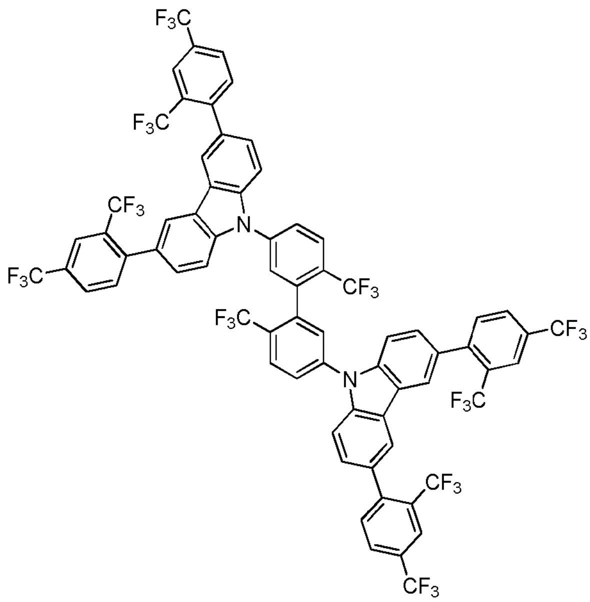

**Figuren**

Es zeigen:

Figur 1 Emissionsspektrum von Beispiel **1** in 10 % PMMA.
Figur 2 Emissionsspektrum von Beispiel **2** in 10 % PMMA.
Figur 3 Emissionsspektrum von Beispiel **3** in 10 % PMMA.
Figur 4 Emissionsspektrum von Beispiel **4** in 10 % PMMA.
Figur 5 Emissionsspektrum von Beispiel **5** in 10 % PMMA.
Figur 6 Emissionsspektrum von Beispiel **6** in 10 % PMMA.

**Patentansprüche**

**1.** Organisches Molekül, aufweisend eine Struktur der Formel I

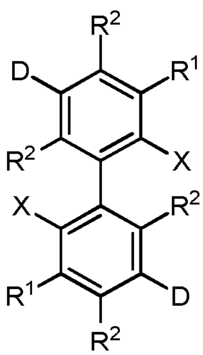

Formel I

mit

X = CN oder CF3, und
D =

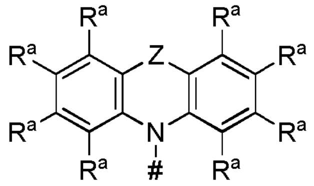

wobei

# ist der Anknüpfungspunkt der Einheit D an einen der in Formel I gezeigten Phenylringe; Z ist eine direkte Bindung oder ausgewählt aus der Gruppe bestehend aus $CR^3R^4$, $C=CR^3R^4$, C=O, $C=NR^3$, $NR^3$, O, $SiR^3R^4$, S, S(O) und $S(O)_2$;

$R^1$ und $R^2$ bei jedem Auftreten gleich oder verschieden ausgewählt ist aus der Gruppe bestehend aus:

- H, Deuterium;
- einer linearen Alkylgruppe mit 1 bis 5 C-Atomen, wobei ein oder mehrere H-Atome durch Deuterium ersetzt sein können;
- einer linearen Alkenyl- oder Alkinylgruppe mit 2 bis 8 C-Atomen, wobei ein oder mehrere H-Atome durch Deuterium ersetzt sein können;
- einer verzweigten oder cyclischen Alkyl-, Alkenyl- oder Alkinylgruppe mit 3 bis 10 C-Atomen, wobei ein oder mehrere H-Atome durch Deuterium ersetzt sein können; und
- einem aromatischen Ringsystem mit 5 bis 15 aromatischen Ringatomen, das jeweils mit einem oder mehreren Resten $R^6$ substituiert sein kann;

$R^a$, $R^3$ und $R^4$ bei jedem Auftreten gleich oder verschieden ausgewählt ist aus der Gruppe bestehend aus:

- H, Deuterium, $N(R^5)_2$, OH, $Si(R^5)_3$, $B(OR^5)_2$, $OSO_2R^5$, $CF_3$, CN, F, Br, I,
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann;

$R^5$ bei jedem Auftreten gleich oder verschieden ausgewählt ist aus der Gruppe bestehend aus:

- H, Deuterium, $N(R^6)_2$, OH, $Si(R^6)_3$, $B(OR^6)_2$, $OSO_2R^6$, $CF_3$, CN, F, Br, I,
- einer linearen Alkyl-, Alkoxy- oder Thioalkoxygruppe mit 1 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$,

C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^6$ substituiert sein kann;
- einer Aryloxy- oder Heteroaryloxygruppe mit 5 bis 60 aromatischen Ringatomen, die mit einem oder mehreren Resten $R^6$ substituiert sein kann, und
- einer Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann;

$R^6$ bei jedem Auftreten gleich oder verschieden ausgewählt ist aus der Gruppe bestehend aus:

- H, Deuterium, OH, $CF_3$, CN, F, Br, I,
- einer linearen Alkyl-, Alkoxy- oder Thioalkoxygruppe mit 1 bis 5 C-Atomen, wobei ein oder mehrere H-Atome durch Deuterium, CN, $CF_3$ oder $NO_2$ ersetzt sein können;
- einer linearen Alkenyl- oder Alkinylgruppe mit 2 bis 5 C-Atomen, wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen;
- einer Aryloxy- oder Heteroaryloxygruppe mit 5 bis 60 aromatischen Ringatomen; und
- einer Diarylaminogruppe, Diheteroarylaminogruppe oder Arylheteroarylaminogruppe mit 10 bis 40 aromatischen Ringatomen;

wobei jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoannelliertes Ringsystem bilden kann; und
wobei mindestens ein $R^a$ ungleich H ist; und
wobei mindestens ein $R^2$ gleich H ist.

2. Organisches Molekül nach Anspruch 1, wobei $R^1$ gleich H oder Methyl und $R^2$ gleich H ist.

3. Organisches Molekül nach Anspruch 1 oder 2, wobei beide X gleich CN sind.

4. Organisches Molekül nach Anspruch 1 bis 3, wobei D eine Struktur der Formel IIa aufweist:

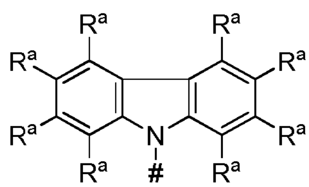

Formel IIa

wobei für# und $R^a$ die in Anspruch 1 genannten Definitionen gelten.

5. Organisches Molekül nach Anspruch 1 bis 4, wobei D eine Struktur der Formel IIb aufweist:

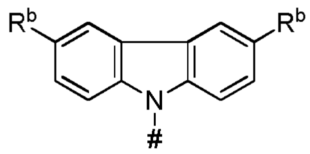

Formel IIb

wobei

$R^b$ bei jedem Auftreten gleich oder verschieden ausgewählt ist aus der Gruppe bestehend aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann;

und für # und $R^5$ die in Anspruch 1 genannten Definitionen gelten.

**6.** Organisches Molekül nach Anspruch 1 bis 4, wobei D eine Struktur der Formel IIc aufweist:

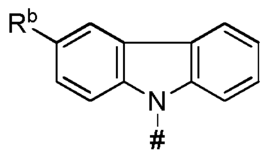

Formel IIc

wobei

$R^b$ bei jedem Auftreten gleich oder verschieden ausgewählt ist aus der Gruppe bestehend aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren

Resten $R^5$ substituiert sein kann, wobei eine oder mehrere nicht benachbarte $CH_2$-Gruppen durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann;

und im Übrigen die in Anspruch 1 genannte Definitionen gelten.

**7.** Organisches Molekül nach Anspruch 5 oder 6, wobei $R^b$ bei jedem Auftreten gleich oder verschieden ausgewählt ist aus der Gruppe bestehend aus:

- Me, $^i$Pr, $^t$Bu, CN, $CF_3$;
- Ph, das jeweils mit einem oder mehreren Resten ausgewählt aus Me, $^i$Pr, $^t$Bu, CN, $CF_3$, oder Ph substituiert sein kann;
- Pyridinyl, das jeweils mit einem oder mehreren Resten ausgewählt aus Me, $^i$Pr, $^t$Bu, CN, $CF_3$, oder Ph substituiert sein kann;
- Pyrimidinyl, das jeweils mit einem oder mehreren Resten ausgewählt aus Me, $^i$Pr, $^t$Bu, CN, $CF_3$, oder Ph substituiert sein kann;
- Carbazolyl, das jeweils mit einem oder mehreren Resten ausgewählt aus Me, $^i$Pr, $^t$Bu, CN, $CF_3$, oder Ph substituiert sein kann; und
- $N(Ph)_2$.

**8.** Verfahren zur Herstellung eines Organischen Moleküls nach Anspruch 1 bis 7, wobei ein in 6-Position $R^1$-substituiertes und in 3- und 5-Position $R^2$-substituiertes 2-Brom-4-fluorbenzonitril oder ein in 6-Position $R^1$-substituiertes und in 3- und 5-Position $R^2$-substituiertes 2-Brom-4-fluorbenzotrifluorid als Edukt eingesetzt wird.

**9.** Verwendung eines organischen Moleküls nach Anspruch 1 bis 7 als lumineszierender Emitter und/oder als Hostmaterial und/oder als Elektronentransportmaterial und/oder als Lochinjektionsmaterial und/oder als Lochblockiermaterial in einer organischen optoelektronischen Vorrichtung.

**10.** Verwendung nach Anspruch 9, wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**11.** Zusammensetzung aufweisend oder bestehend aus:

(a) mindestens einem organischen Molekül nach einem der Ansprüche 1 bis 7, insbesondere als Emitter und/oder Host, und
(b) ein oder mehrere von dem Molekül nach einem der Ansprüche 1 bis 7 verschiedenen Emitter- und/oder

Hostmaterialien und
(c) optional einem oder mehreren Farbstoffen und/ oder einem oder mehreren Lösungsmitteln.

**12.** Organische optoelektronische Vorrichtung, aufweisend ein organisches Molekül nach Anspruch 1 bis 7 oder eine Zusammensetzung nach Anspruch 11, insbesondere ausgeformt als eine Vorrichtung ausgewählt aus der Gruppe bestehend ausorganischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**13.** Organische optoelektronische Vorrichtung nach Anspruch 12, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein organisches Molekül nach Anspruch 1 bis 7 oder eine Zusammensetzung nach Anspruch 11 aufweist.

**14.** Verfahren zur Herstellung eines optoelektronischen Bauelements, wobei ein organisches Molekül nach Anspruch 1 bis 7 verwendet wird.

**15.** Verfahren nach Anspruch 14, umfassend die Verarbeitung des organischen Moleküls mittels eines Vakuumverdampfungsverfahrens oder aus einer Lösung.

## Claims

**1.** Organic molecule, comprising a structure of Formula I

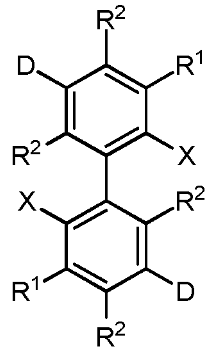

Formula I

with

X = CN or $CF_3$, and
D =

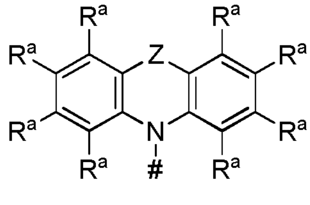

wherein
# is the point of attachment of unit D to one of the phenyl rings shown in Formula I;
Z is a direct bond or is selected from the group consisting of $CR^3R^4$, $C=CR^3R^4$, C=O, $C=NR^3$, $NR^3$, O, $SiR^3R^4$,

S, S(O) and $S(O)_2$;
in each occurrence $R^1$ and $R^2$ are the same or different and are selected from the group consisting of:

- H, deuterium;
- a linear alkyl group having 1 to 5 C atoms, wherein one or more H atoms can be replaced by deuterium;
- a linear alkenyl or alkynyl group having 2 to 8 C atoms, wherein one or more H atoms can be replaced by deuterium;
- a branched or cyclic alkyl, alkenyl or alkynyl group having 3 to 10 C atoms, wherein one or more H atoms can be replaced by deuterium; and
- -an aromatic ring system having 5 to 15 aromatic ring atoms, which can in each case be substituted with one or more radicals $R^6$;

in each occurrence $R^a$, $R^3$ and $R^4$ are the same or different and are selected from the group consisting of:

- H, deuterium, $N(R^5)_2$, OH, $Si(R^5)_3$, $B(OR^5)_2$, $OSO_2R^5$, $CF_3$, CN, F, Br, I,
- a linear alkyl, alkoxy or thioalkoxy group having 1 to 40 C atoms, which can in each case can be substituted with one or more radicals $R^5$, wherein one or more non-adjacent $CH_2$ groups can be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- a linear alkenyl or alkynyl group having 2 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- a branched or cyclic alkyl, alkenyl, alkynyl, alkoxy or thioalkoxy group having 3 to 40 C atoms, which can in each case be substituted with one or more radicals $R^5$, wherein one or more non-adjacent $CH_2$ groups can be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- an aromatic or heteroaromatic ring system having 5 to 60 aromatic ring atoms, which can in each case be substituted with one or more radicals $R^5$;
- an aryloxy or heteroaryloxy group having 5 to 60 aromatic ring atoms, which can be substituted with one or more radicals $R^5$; and
- a diarylamino group, diheteroarylamino group or arylheteroarylamino group having 10 to 40 aromatic ring atoms, which can be substituted with one or more radicals $R^5$;

in each occurrence $R^5$ is the same or different and is selected from the group consisting of:

- H, deuterium, $N(R^6)_2$, OH, $Si(R^6)_3$, $B(OR^6)_2$, $OSO_2R^6$, $CF_3$, CN, F, Br, I,
- a linear alkyl, alkoxy or thioalkoxy group having 1 to 40 C atoms, which can in each case be substituted with one or more radicals $R^6$, wherein one or more non-adjacent $CH_2$ groups can be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms can be replaced by deuterium, CN, $CF_3$ or $NO_2$;
- a linear alkenyl or alkynyl group having 2 to 40 C atoms, which can in each case be substituted with one or more radicals $R^6$, wherein one or more non-adjacent $CH_2$ groups can be replaced by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms can be replaced by deuterium, CN, $CF_3$ or $NO_2$;
- a branched or cyclic alkyl, alkenyl, alkynyl, alkoxy or thioalkoxy group having 3 to 40 C atoms, which can in each case be substituted with one or more radicals $R^6$, wherein one or more non-adjacent $CH_2$ groups can be replaced by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms can be replaced by deuterium, CN, $CF_3$ or $NO_2$;
- an aromatic or heteroaromatic ring system having 5 to 60 aromatic ring atoms, which can in each case be substituted with one or more radicals $R^6$;
- an aryloxy or heteroaryloxy group having 5 to 60 aromatic ring atoms, which can be substituted with one or more radicals $R^6$; and
- a diarylamino group, diheteroarylamino group or arylheteroarylamino group having 10 to 40 aromatic ring atoms, which can be substituted with one or more radicals $R^6$;

in each occurrence $R^6$ is the same or different and is selected from the group consisting of:

- H, deuterium, OH, $CF_3$, CN, F, Br, I,
- a linear alkyl, alkoxy or thioalkoxy group having 1 to 5 C atoms, wherein one or more H atoms can be replaced by deuterium, CN, $CF_3$ or $NO_2$;
- a linear alkenyl or alkynyl group having 2 to 5 C atoms, wherein one or more H atoms can be replaced by deuterium, CN, $CF_3$ or $NO_2$;
- a branched or cyclic alkyl, alkenyl, alkynyl, alkoxy or thioalkoxy group having 3 to 5 C atoms, wherein one or more H atoms can be replaced by deuterium, CN, $CF_3$ or $NO_2$;
- an aromatic or heteroaromatic ring system having 5 to 60 aromatic ring atoms;
- an aryloxy or heteroaryloxy group having 5 to 60 aromatic ring atoms; and
- a diarylamino group, diheteroarylamino group or arylheteroarylamino group having 10 to 40 aromatic ring atoms;

wherein each of the radicals $R^a$, $R^3$, $R^4$ or $R^5$ can also form a mono- or polycyclic, aliphatic, aromatic and/or benzoannelated ring system with one or more further radicals $R^a$, $R^3$, $R^4$ or $R^5$; and
wherein at least one $R^a$ is not H, and
wherein at least one $R^2$ is H.

**2.** Organic molecule according to Claim 1, wherein $R^1$ is H or methyl and $R^2$ is H.

**3.** Organic molecule according to Claim 1 or 2, wherein both X are CN.

**4.** Organic molecule according to Claim 1 to 3, wherein D has a structure of Formula IIa:

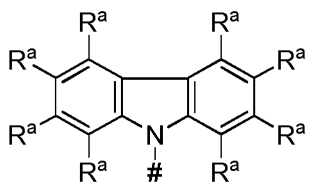

Formula IIa

wherein the definitions mentioned in Claim 1 apply for # and $R^a$.

**5.** Organic molecule according to Claim 1 to 4, wherein D has a structure of Formula IIb:

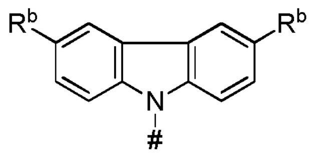

Formula IIb

wherein

in each occurrence $R^b$ is the same or different and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group having 1 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- a linear alkenyl or alkynyl group having 2 to 40 C atoms, which can in each case be substituted with one or more radicals $R^5$, wherein one or more non-adjacent $CH_2$ groups can be replaced by $R^5C=CR^5$, C≡C, $Si(R5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- a branched or cyclic alkyl, alkenyl, alkynyl, alkoxy or thioalkoxy group having 3 to 40 C atoms, which can

in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- an aromatic or heteroaromatic ring system having 5 to 60 aromatic ring atoms, which can in each case be substituted with one or more radicals $R^5$;
- an aryloxy or heteroaryloxy group having 5 to 60 aromatic ring atoms, which can be substituted with one or more radicals $R^5$; and
- a diarylamino group, diheteroarylamino group or arylheteroarylamino group having 10 to 40 aromatic ring atoms, which can be substituted with one or more radicals $R^5$;

and the definitions mentioned in Claim 1 apply for # and $R^5$.

**6.** Organic molecule according to Claim 1 to 4, wherein D comprises a structure of Formula IIc:

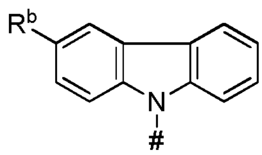

Formula IIc

wherein

in each occurrence $R^b$ is the same or different and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group having 1 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- a linear alkenyl or alkynyl group having 2 to 40 C atoms, which can in each case be substituted with one or more radicals $R^5$, wherein one or more non-adjacent $CH_2$ groups can be replaced by $R^5C=CR^5$, C≡C, Si(R5)2, Ge(R5)2, Sn(R5)2, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- a branched or cyclic alkyl, alkenyl, alkynyl, alkoxy or thioalkoxy group having 3 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$;
- an aromatic or heteroaromatic ring system having 5 to 60 aromatic ring atoms, which can in each case be substituted with one or more radicals $R^5$;
- an aryloxy or heteroaryloxy group having 5 to 60 aromatic ring atoms, which can be substituted with one or more radicals $R^5$; and
- a diarylamino group, diheteroarylamino group or arylheteroarylamino group having 10 to 40 aromatic ring atoms, which can be substituted with one or more radicals $R^5$;

and otherwise the definitions mentioned in Claim 1 apply.

**7.** Organic molecule according to Claim 5 or 6, wherein in each occurrence $R^b$ is the same or different and is selected from the group consisting of:

- Me, $^i$Pr, $^t$Bu, CN, $CF_3$;
- Ph, which can in each case be substituted with one or more radicals selected from Me, $^i$Pr, $^t$Bu, CN, $CF_3$, or Ph;
- pyridinyl, which can in each case be substituted with one or more radicals selected from Me, $^i$Pr,$^t$Bu, CN, $CF_3$, or Ph;
- pyrimidinyl which can in each case be substituted with one or more radicals selected from Me, $^i$Pr, $^t$Bu, CN, $CF_3$, or Ph;
- carbazolyl which can in each case be substituted with one or more radicals selected from Me, $^i$Pr, $^t$Bu, CN,

$CF_3$, or Ph; and
- $N(Ph)_2$.

8. Method for producing an organic molecule according to Claims 1 to 7, wherein a in 6 position $R^1$-substituted and in 3 and 5 position $R^2$-substituted 2-bromo-4-fluorobenzonitrile or a in 6 position $R^1$-substituted and in 3 and 5 position $R^2$-substituted 2-bromo-4-fluorobenzotrifluoride is used as an educt.

9. Use of an organic molecule according to Claims 1 to 7 as a luminescent emitter and/or as a host material and/or as an electron transport material and/or as a hole injection material and/or as a hole blocking material in an organic optoelectronic device.

10. Use according to Claim 9, wherein the organic optoelectronic device is selected from the group consisting of:

• organic light-emitting diodes (OLEDs),
• light-emitting electrochemical cells,
• OLED sensors, in particular in gas and vapor sensors which are not hermetically shielded to the outside,
• organic diodes,
• organic solar cells,
• organic transistors,
• organic field-effect transistors,
• organic lasers and
• down-conversion elements.

11. Composition comprising or consisting of:

(a) at least one organic molecule according to any one of Claims 1 to 7, in particular as an emitter and/or host, and
(b) one or more emitter and/or host materials different from the molecule according to any one of Claims 1 to 7, and
(c) optionally one or more dyes and/or one or more solvents.

12. Organic optoelectronic device comprising an organic molecule according to Claim 1 to 7 or a composition according to Claim 11, in particular formed as a device selected from the group consisting of organic light-emitting diode (OLED), light-emitting electrochemical cell, OLED sensor, in particular gas and vapor sensors which are not hermetically shielded to the outside, organic diode, organic solar cell, organic transistor, organic field-effect transistor, organic laser and down-conversion element.

13. Organic optoelectronic device according to Claim 12, comprising

- a substrate,
- an anode and
- a cathode, wherein the anode or the cathode are disposed on the substrate, and
- at least one light-emitting layer, which is disposed between the anode and the cathode and has the organic molecule according to Claim 1 to 7 or a composition according to Claim 11.

14. Method for producing an optoelectronic component, wherein an organic molecule according to Claim 1 to 7 is used.

15. Method according to Claim 14, comprising the processing of the organic molecule by means of a vacuum evaporation method or from a solution.

## Revendications

1. Molécule organique, comprenant une structure de formule I

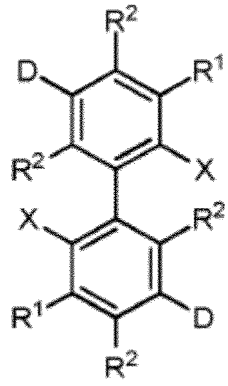

formule I

avec

X = CN ou $CF_3$, et
D =

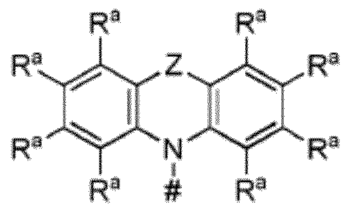

# étant le point de fixation du motif D à l'un des cycles phényle montrés dans la formule I ;
Z étant une liaison directe ou étant choisi dans le groupe constitué par $CR^3R^4$, $C=CR^3R^4$, C=O, $C=NR^3$, $NR^3$, O, $SiR^3R^4$, S, S(O) et $S(O)_2$ ;
$R^1$ et $R^2$, identiques ou différents en chaque occurrence, étant choisis dans le groupe constitué par :

- H, deutérium ;
- un groupe alkyle linéaire comportant 1 à 5 atomes de C, un ou plusieurs atomes de H pouvant être remplacés par deutérium ;
- un groupe alcényle ou alcynyle linéaire comportant 2 à 8 atomes de C, un ou plusieurs atomes de H pouvant être remplacés par deutérium ;
- un groupe alkyle, alcényle ou alcynyle, ramifié ou cyclique, comportant 3 à 10 atomes de C, un ou plusieurs atomes de H pouvant être remplacés par deutérium ; et
- un système cyclique aromatique comportant 5 à 15 atomes de cycle aromatique, qui peut être à chaque fois substitué par un ou plusieurs radicaux $R^6$ ;

$R^a$, $R^3$ et $R^4$, identiques ou différents en chaque occurrence, étant choisis dans le groupe constitué par :

- H, deutérium, $N(R^5)_2$, OH, $Si(R^5)_3$, $B(OR^5)_2$, $OSO_2R^5$, $CF_3$, CN, F, Br, I,
- un groupe alkyle, alcoxy ou thioalcoxy comportant 1 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$ ;
- un groupe alcényle ou alcynyle linéaire comportant 2 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$ ;
- un groupe alkyle, alcényle, alcynyle, alcoxy ou thioalcoxy, ramifié ou cyclique, comportant 3 à 40 atomes de C, qui peut être à chaque fois substitué par un ou plusieurs radicaux $R^5$, un ou plusieurs groupes $CH_2$

non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique, qui peut être à chaque fois substitué par un ou plusieurs radicaux $R^5$ ;
- un groupe aryloxy ou hétéroaryloxy comportant 5 à 60 atomes de cycle aromatique, qui peut être substitué par un ou plusieurs radicaux $R^5$; et
- un groupe diarylamino, un groupe dihétéroarylamino ou un groupe arylhétéroarylamino comportant 10 à 40 atomes de cycle aromatique, qui peut être substitué par un ou plusieurs radicaux $R^5$ ;

$R^5$, identique ou différent en chaque occurrence, étant choisi dans le groupe constitué par :

- H, deutérium, $N(R^6)_2$, OH, $Si(R^6)_3$, $B(OR^6)_2$, $OSO_2R^6$, $CF_3$, CN, F, Br, I,
- un groupe alkyle, alcoxy ou thioalcoxy comportant 1 à 40 atomes de C, qui peut être à chaque fois substitué par un ou plusieurs radicaux $R^6$, un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$ et un ou plusieurs atomes de H pouvant être remplacés par deutérium, CN, $CF_3$ ou $NO_2$ ;
- un groupe alcényle ou alcynyle linéaire comportant 2 à 40 atomes de C, qui peut être à chaque fois substitué par un ou plusieurs radicaux $R^6$, un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$ et un ou plusieurs atomes de H pouvant être remplacés par deutérium, CN, $CF_3$ ou $NO_2$ ;
- un groupe alkyle, alcényle, alcynyle, alcoxy ou thioalcoxy, ramifié ou cyclique, comportant 3 à 40 atomes de C, qui peut être à chaque fois substitué par un ou plusieurs radicaux $R^6$, un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$ et un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique, qui peut être à chaque fois substitué par un ou plusieurs radicaux $R^6$ ;
- un groupe aryloxy ou hétéroaryloxy comportant 5 à 60 atomes de cycle aromatique, qui peut être substitué par un ou plusieurs radicaux $R^6$ ; et
- un groupe diarylamino, un groupe dihétéroarylamino ou un groupe arylhétéroarylamino comportant 10 à 40 atomes de cycle aromatique, qui peut être substitué par un ou plusieurs radicaux $R^6$ ;

$R^6$, identique ou différent en chaque occurrence, étant choisi dans le groupe constitué par :

- H, deutérium, OH, $CF_3$, CN, F, Br, I,
- un groupe alkyle, alcoxy ou thioalcoxy linéaire comportant 1 à 5 atomes de C, un ou plusieurs atomes de H pouvant être remplacés par deutérium, CN, $CF_3$ ou $NO_2$ ;
- un groupe alcényle ou alcynyle linéaire comportant 2 à 5 atomes de C, un ou plusieurs atomes de H pouvant être remplacés par deutérium, CN, $CF_3$ ou $NO_2$ ;
- un groupe alkyle, alcényle, alcynyle, alcoxy ou thioalcoxy, ramifié ou cyclique, comportant 3 à 5 atomes de C, un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique ;
- un groupe aryloxy ou hétéroaryloxy comportant 5 à 60 atomes de cycle aromatique ; et
- un groupe diarylamino, un groupe dihétéroarylamino ou un groupe arylhétéroarylamino comportant 10 à 40 atomes de cycle aromatique ;

chacun des radicaux $R^a$, $R^3$, $R^4$ ou $R^5$ pouvant également former un système cyclique monocyclique ou polycyclique, aliphatique, aromatique et/ou benzoannelé avec un ou plusieurs autres radicaux $R^a$, $R^3$, $R^4$ ou $R^5$; et
au moins un $R^a$ étant différent de H ; et
au moins un $R^2$ étant égal à H.

**2.** Molécule organique selon la revendication 1, $R^1$ étant égal à H ou méthyle et $R^2$ étant égal à H.

**3.** Molécule organique selon la revendication 1 ou 2, les deux X étant égaux à CN.

**4.** Molécule organique selon les revendications 1 à 3, D comprenant une structure de formule IIa :

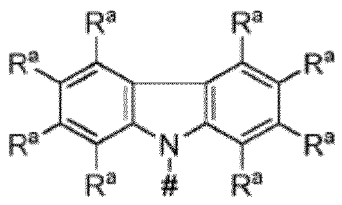

formule IIa

les définitions mentionnées dans la revendication 1 s'appliquant pour # et $R^a$.

**5.** Molécule organique selon les revendications 1 à 4, D comprenant une structure de formule IIb :

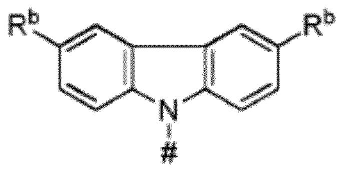

formule IIb

$R^b$, identique ou différent en chaque occurrence, étant choisi dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy comportant 1 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$ ;
- un groupe alcényle ou alcynyle linéaire comportant 2 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$ ;
- un groupe alkyle, alcényle, alcynyle, alcoxy ou thioalcoxy, ramifié ou cyclique, comportant 3 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique, qui peut être à chaque fois substitué par un ou plusieurs radicaux $R^5$ ;
- un groupe aryloxy ou hétéroaryloxy comportant 5 à 60 atomes de cycle aromatique, qui peut être substitué par un ou plusieurs radicaux $R^5$; et
- un groupe diarylamino, un groupe dihétéroarylamino ou un groupe arylhétéroarylamino comportant 10 à 40 atomes de cycle aromatique, qui peut être substitué par un ou plusieurs radicaux $R^5$ ;

les définitions mentionnées dans la revendication 1 s'appliquant pour # et $R^5$.

**6.** Molécule organique selon les revendications 1 à 4, D comprenant une structure de formule IIc :

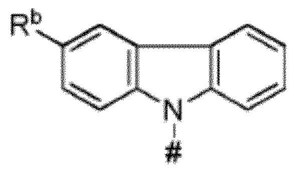

formule IIc

$R^b$, identique ou différent en chaque occurrence, étant choisi dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy comportant 1 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$;
- un groupe alcényle ou alcynyle linéaire comportant 2 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$;
- un groupe alkyle, alcényle, alcynyle, alcoxy ou thioalcoxy, ramifié ou cyclique, comportant 3 à 40 atomes de C, qui peut être à chaque fois substitué par un ou plusieurs radicaux $R^5$, un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ et un ou plusieurs atomes de H pouvant être remplacés par deutérium, CN, $CF_3$ ou $NO_2$ ;
- un système cyclique aromatique ou hétéroaromatique comportant 5 à 60 atomes de cycle aromatique, qui peut être à chaque fois substitué par un ou plusieurs radicaux $R^5$ ;
- un groupe aryloxy ou hétéroaryloxy comportant 5 à 60 atomes de cycle aromatique, qui peut être substitué par un ou plusieurs radicaux $R^5$; et
- un groupe diarylamino, un groupe dihétéroarylamino ou un groupe arylhétéroarylamino comportant 10 à 40 atomes de cycle aromatique, qui peut être substitué par un ou plusieurs radicaux $R^5$ ;

et pour le reste, les définitions mentionnées dans la revendication 1 s'appliquant.

**7.** Molécule organique selon la revendication 5 ou 6, $R^b$, identique ou différent en chaque occurrence, étant choisi dans le groupe constitué par :

- Me, $^i$Pr, $^t$Bu, CN, $CF_3$ ;
- Ph, qui peut à chaque fois être substitué par un ou plusieurs radicaux choisis parmi Me, $^i$Pr, $^t$Bu, CN, $CF_3$ et Ph ;
- pyridinyle, qui peut à chaque fois être substitué par un ou plusieurs radicaux choisis parmi Me, $^i$Pr, $^t$Bu, CN, $CF_3$ et Ph ;
- pyrimidinyle, qui peut à chaque fois être substitué par un ou plusieurs radicaux choisis parmi Me, $^i$Pr, $^t$Bu, CN, $CF_3$ et Ph ;
- carbazolyle, qui peut à chaque fois être substitué par un ou plusieurs radicaux choisis parmi Me, $^i$Pr, $^t$Bu, CN, $CF_3$ et Ph ; et
- $N(Ph)_2$.

**8.** Procédé pour la préparation d'une molécule organique selon les revendications 1 à 7, un 2-bromo-4-fluorobenzonitrile substitué par $R^1$ en position 6 et substitué par $R^2$ en positions 3 et 5 ou un 2-bromo-4-fluorobenzotrifluorure substitué par $R^1$ en position 6 et substitué par $R^2$ en positions 3 et 5 étant utilisé comme éduit.

**9.** Utilisation d'une molécule organique selon les revendications 1 à 7 en tant qu'émetteur et/ou en tant que matériau hôte et/ou en tant que matériau de transport d'électrons et/ou en tant que matériau d'injection de trous et/ou en tant que matériau de blocage de trous, luminescent, dans un dispositif optoélectronique organique.

**10.** Utilisation selon la revendication 9, le dispositif optoélectronique organique étant choisi dans le groupe constitué par :

• des diodes organiques luminescentes (OLED),
• des cellules électrochimiques luminescentes,
• des capteurs OLED, en particulier dans des capteurs de gaz et de vapeur non hermétiquement protégés vers l'extérieur,
• des diodes organiques,
• des cellules solaires organiques,
• des transistors organiques,
• des transistors organiques à effet de champ,
• des lasers organiques, et

• des éléments de conversion par abaissement.

**11.** Composition comprenant ou constituée de :

(a) au moins une molécule organique selon l'une quelconque des revendications 1 à 7, en particulier en tant qu'émetteur et/ou hôte, et
(b) un ou plusieurs matériaux émetteurs et/ou hôtes différents de la molécule selon l'une quelconque des revendications 1 à 7 et
(c) éventuellement un ou plusieurs colorants et/ou un ou plusieurs solvants.

**12.** Dispositif optoélectronique organique, comprenant une molécule organique selon les revendications 1 à 7 ou une composition selon la revendication 11, en particulier sous forme d'un dispositif choisi dans le groupe constitué par une diode luminescente organique (OLED), une cellule électrochimique luminescente, un capteur OLED, en particulier des capteurs de gaz et de vapeur non hermétiquement protégés vers l'extérieur, une diode organique, une cellule solaire organique, un transistor organique, un transistor organique à effet de champ, un laser organique et un élément de conversion par abaissement.

**13.** Dispositif optoélectronique organique selon la revendication 12, présentant

- un substrat,
- une anode et
- une cathode, l'anode ou la cathode étant appliquée sur le substrat, et
- au moins une couche luminescente, qui est disposée entre l'anode et la cathode et qui présente une molécule organique selon les revendications 1 à 7 ou une composition selon la revendication 11.

**14.** Procédé pour la préparation d'un composant optoélectronique, une molécule organique selon les revendications 1 à 7 étant utilisée.

**15.** Procédé selon la revendication 14, comprenant le traitement de la molécule organique au moyen d'un procédé d'évaporation sous vide ou à partir d'une solution.

**Figuren**

**Figur 1**

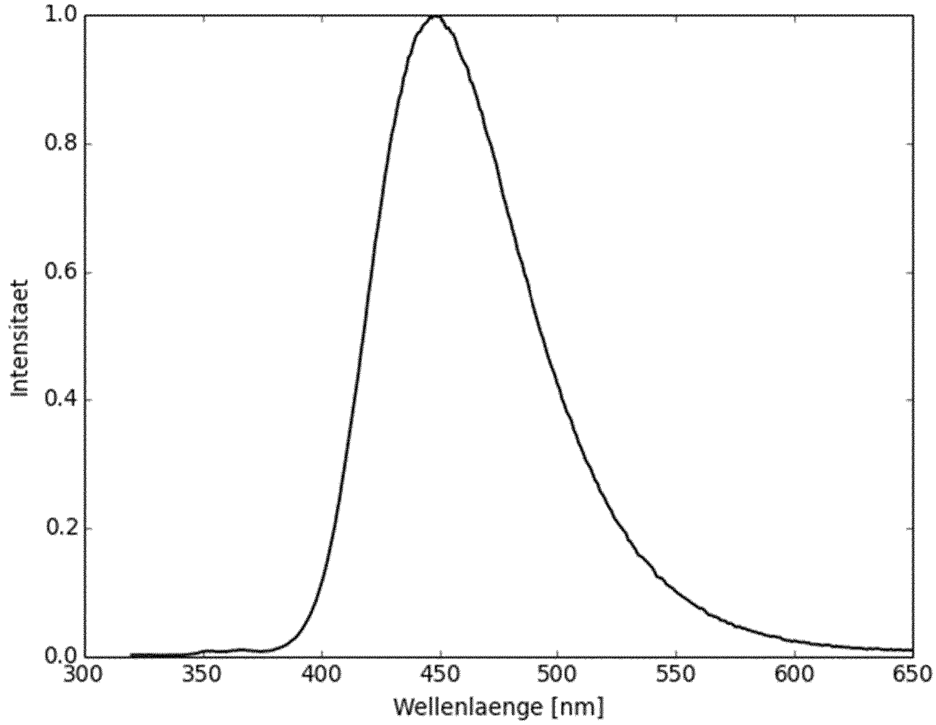


**Figur 2**

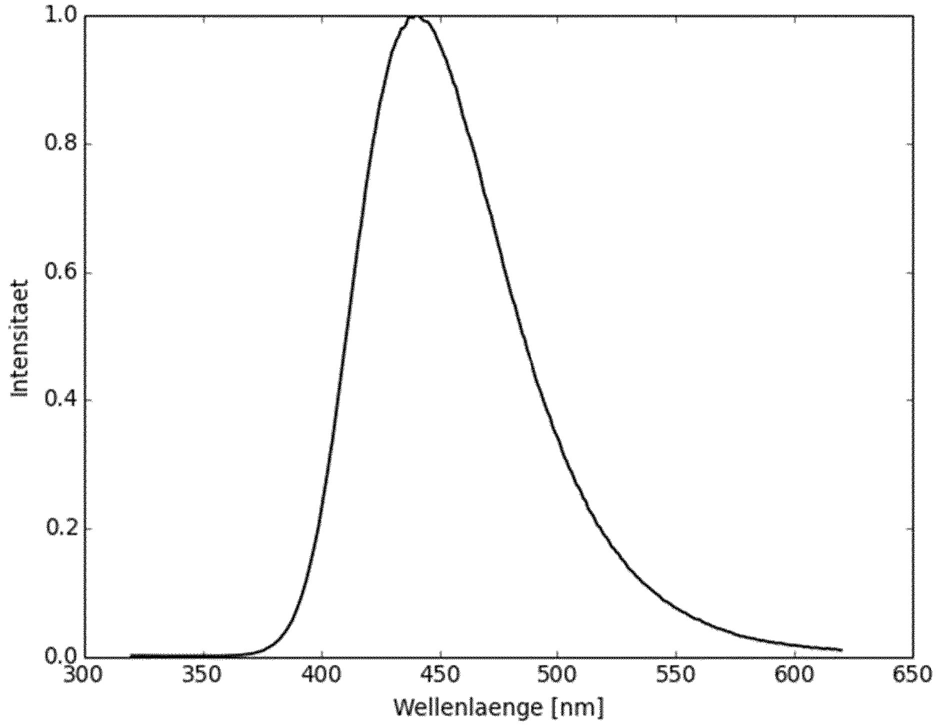

**Figur 3**

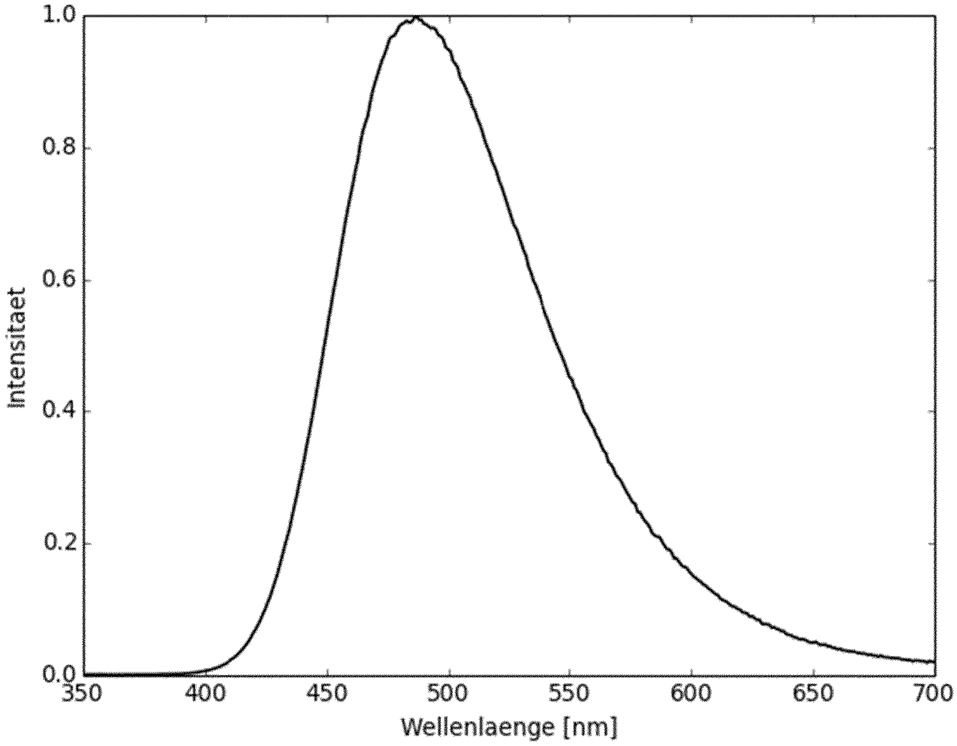


**Figur 4**

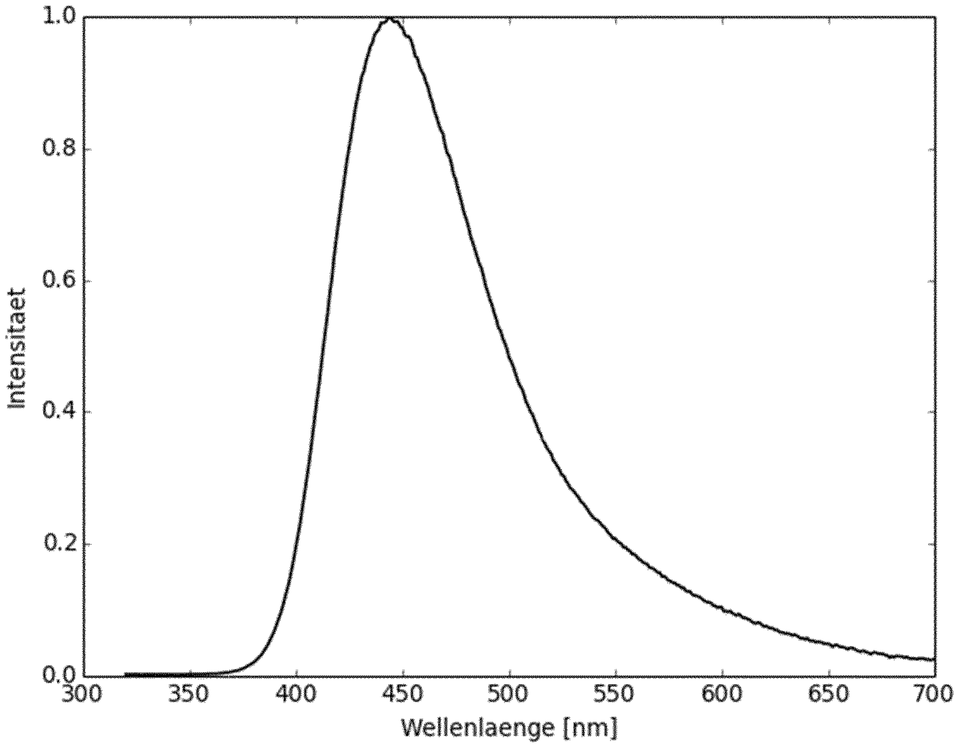

Figur 5

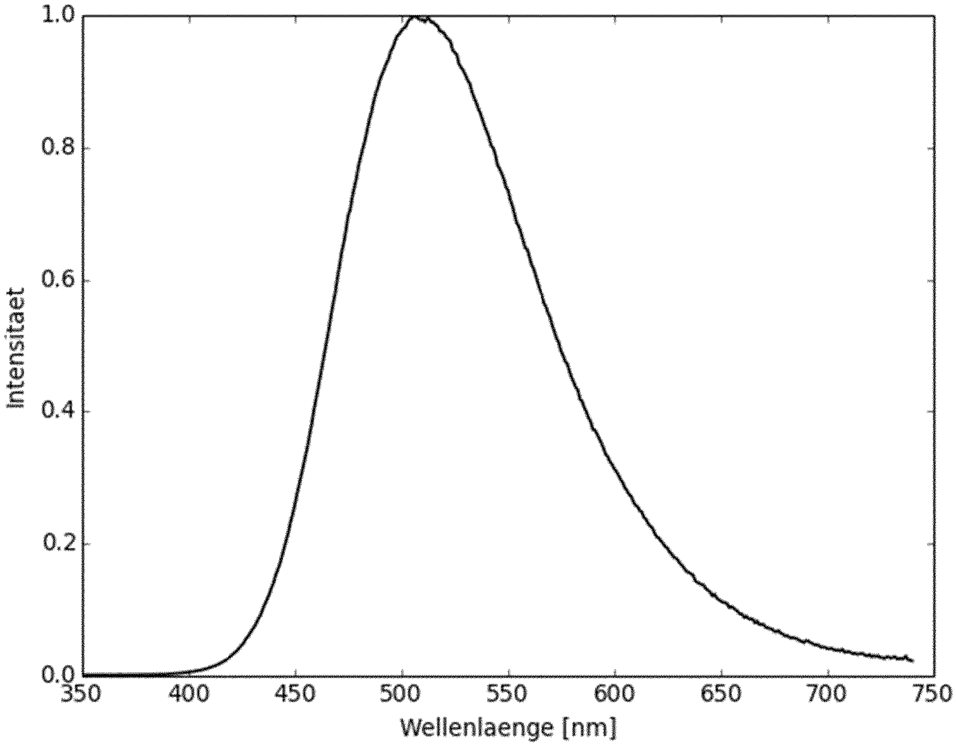


Figur 6

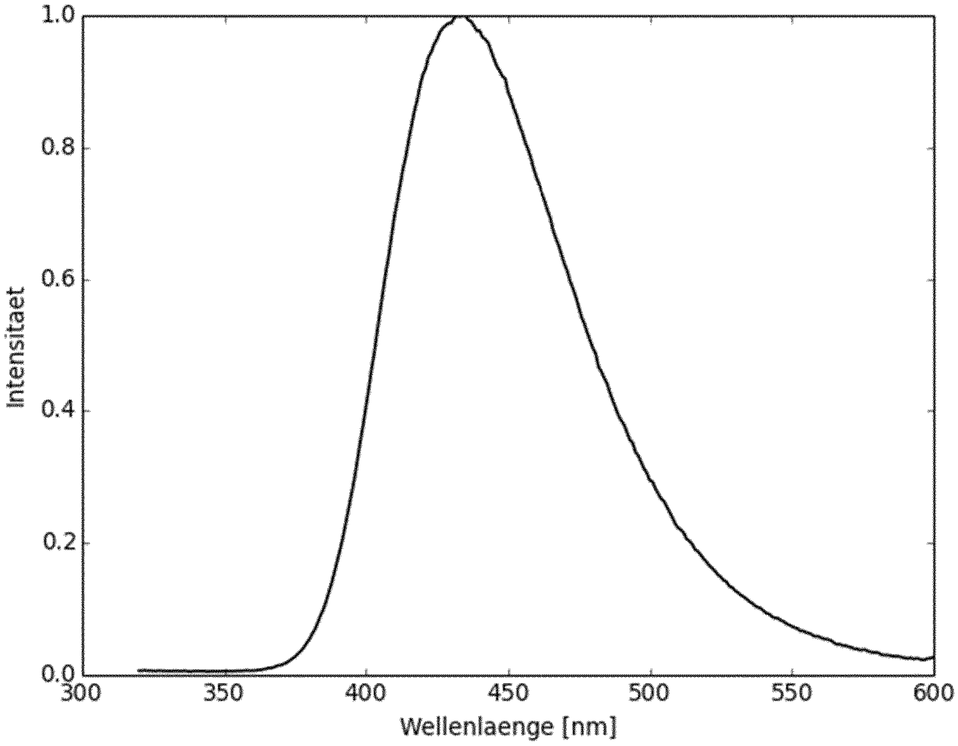

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **B. M.E. THOMPSON et al.** *Chem. Mater.,* 2004, vol. 16, 4743